# EUROPEAN PATENT SPECIFICATION

(11) **EP 3 338 088 B1**
(45) Date of publication and mention of the grant of the patent: **08.01.2025**
(21) Application number: 15901516.3
(22) Date of filing: 20.08.2015
(51) Int. Cl.: G01N 33/50, G01N 33/493, C12Q 1/04

(54) **BIOMARKERS FOR CORONARY HEART DISEASE**
BIOMARKER FÜR KORONARE HERZERKRANKUNG
BIOMARQUEURS DE CORONAROPATHIE

(43) Date of publication of application: 27.06.2018
(73) Proprietor: BGI Shenzhen, Shenzhen, Guangdong 518083 (CN)
(72) Inventor: FENG, Qiang, Shenzhen Guangdong 518083 (CN); LIU, Zhipeng, Shenzhen Guangdong 518083 (CN); WANG, Jun, Shenzhen Guangdong 518083 (CN)
(74) Representative: J A Kemp LLP
(86) International application number: PCT/CN2015/087675
(87) International publication number: WO 2017/028312

(56) References cited:
- WO-A1-2008/031917
- WO-A1-2013/070839
- WO-A1-2014/019271
- CN-A- 104 614 291
- QIANG FENG ET AL: "Integrated metabolomics and metagenomics analysis of plasma and urine identified microbial metabolites associated with coronary heart disease", SCIENTIFIC REPORTS, vol. 6, no. 1, 2 March 2016 (2016-03-02), XP055525251, DOI: 10.1038/srep22525
- JOMAY CHOW ET AL: "Fecal Metabolomics of Healthy Breast-Fed versus Formula-Fed Infants before and during In Vitro Batch Culture Fermentation", JOURNAL OF PROTEOME RESEARCH, vol. 13, no. 5, 2 May 2014 (2014-05-02), pages 2534 - 2542, XP055275560, ISSN: 1535-3893, DOI: 10.1021/pr500011w
- SVATI H. SHAH ET AL: "Metabolomic Profiling for the Identification of Novel Biomarkers and Mechanisms Related to Common Cardiovascular Diseases : Form and Function", CIRCULATION, vol. 126, no. 9, 28 August 2012 (2012-08-28), US, pages 1110 - 1120, XP055525353, ISSN: 0009-7322, DOI: 10.1161/CIRCULATIONAHA.111.060368
- ZENENG WANG ET AL: "Gut flora metabolism of phosphatidylcholine promotes cardiovascular disease", NATURE, vol. 472, no. 7341, 6 April 2011 (2011-04-06), pages 57 - 63, XP055120871, ISSN: 0028-0836, DOI: 10.1038/nature09922
- CLAUDIA NONNENMACHER ET AL: "Periodontal Microbiota in Patients With Coronary Artery Disease Measured by Real-Time Polymerase Chain Reaction: A Case-Control Study", JOURNAL OF PERIODONTOLOGY., vol. 78, no. 9, 1 September 2007 (2007-09-01), US, pages 1724 - 1730, XP055525600, ISSN: 0022-3492, DOI: 10.1902/jop.2007.060345
- ANDREA GANNA ET AL: "Large-scale Metabolomic Profiling Identifies Novel Biomarkers for Incident Coronary Heart Disease", PLOS GENETICS, vol. 10, no. 12, 11 December 2014 (2014-12-11), pages 1 - 10, XP055363370, DOI: 10.1371/journal.pgen.1004801
- BROWN, C.T. ET AL.: "Genome resolved analysis of a premature infant gut microbial community reveals a Varibaculum cambriense genome and a shift towards fermentation-based metabolism during the third week of life", MICROBIOME, vol. 1, no. 30, 17 December 2013 (2013-12-17), pages 1 - 19, XP021173860
- HAHNE, H. ET AL.: "Discovery of O-GlcNAc-6-phosphate Modified Proteins in Large-scale Phosphoproteomics Data", MOLECULAR & CELLULAR PROTEOMICS, vol. 11, no. 10, 23 July 2012 (2012-07-23), pages 1063 - 1069, XP055364580
- DAVID, J.: "How Industry Uses Big Data:Metagenomics and Beyond", 119TH AFDO ANNUAL EDUCATIONAL CONFERENCE, 22 June 2015 (2015-06-22), pages 1 - 25, XP055466555
- GRIFFITHS, W.J. ET AL.: "Targeted Metabolomics for Biomarker Disvovery", ANGEW. CHEM. INT.ED., vol. 49, no. 32, 26 July 2010 (2010-07-26), pages 5426 - 5445, XP055364583

## Description

### FIELD

The present invention relates to biomarkers and methods for predicting the risk of a disease related to metabolites, in particular coronary heart disease.

### BACKGROUND

Coronary heart disease (CHD) is the top risk factor in modern society with annual mortality rate overpassing the sum of all types of cancers. The majority of cardiovascular deaths occurrence are related to the extent of people's awareness of their own medical conditions and are due to lackness of in-time treatment as demonstrated by a five-year follow-up study by MaGiCAD cohort (Graninger, D.J. & Mosedale, D.E. Metabolomics in coronary heart disease.Heart.Metab.55, 8-12 (2012)). The challenge for early diagnosis and prevention of CHD lies in the lackness of reliable non-invasive biomarkers. The "gold standard" for diagnosis of CHD is still coronary angiography which is invasive and accompanied by many deadly side effects, this limited the large population screening and the CHD risk prediction at early stage.

Many researches point the fatty acids play important roles in the heart metabolism; they are predominant substrates, accounting for 60-90% cardiac ATP synthesis, for cardiac ATP generation by mitochondrial oxidative phosphorylation under normal physiological conditions. Cardiovascular diseases (CVD) like coronary heart disease and cardiac failure undergo a "metabolic shift" as a consequence of both intrinsic and extrinsic perturbations. Increased low-density lipoprotein cholesterol (LDL-C) has previously been considered as one of the major risk factors for CHD. The fact that core defects in cardiovascular disease are lipids metabolism (Fernandez, C. et al. Plasma lipid composition and risk of developing cardiovascular disease. PLoS ONE 8, e71846 (2013)) makes metabolomics a particularly promising method to study these types of diseases.

Metabolomics is an innovative and high-throughput bioanalytical method aiming to identify and quantify small molecules (molecular weight less than 1500 Daltons) present in any biological system or any specific physiological state. Two major analytical techniques, nuclear magnetic resonance (NMR) and mass spectrometry (MS), have been widely used in endogenous compounds measurement at an exponential increasing rate in last decade. MS-based techniques have made rapid progress and have been used more frequently compared with NMR since 2005 because of the following advantages: higher sensitivity, more coverage of the metabolome, improved metabolites identification and discrimination capability, and modularity to perform compound-class-specific analysis (Griffiths, W.J. et al. Targeted metabolomics for biomarker discovery. Angew.Chem. Int. Ed. 49, 5426-5445 (2010)). MS is mostly used in conjunction with chromatography, such as gas chromatography mass spectrometry (GC-MS) and liquid chromatography mass spectrometry (LC-MS). Svati H. Shah et al., (2012), Circulation, vol. 126, no. 9, pages 1110-1120, describes metabolomic profiling for the identification of novel biomarkers and mechanisms related to common cardiovascular diseases. Zeneng Wang et al., (2011), Nature, vol. 472, no. 7341, pages 57-63, discloses that gut flora metabolism of phosphatidylcholine promotes cardiovascular disease. Claudia Nonnenmacher et al., (2007), Journal of Periodontology, vol. 78, no. 9, pages 1724-1730 describes periodontal microbiota in patients with coronary artery disease measured by Real-Time Polymerase Chain Reaction. Andrea Ganna et al., (2014), PLOS Genetics, vol. 10, no. 12, pages 1-10 discloses that large-scale metabolomic profiling identifies novel biomarkers for incident coronary heart disease.

### SUMMARY OF THE INVENTION

The present invention provides a method for diagnosis of CHD, and specifically comprises the following aspects.

The present invention provides a method for diagnosis of CHD in a subject, wherein the method comprises the following steps: 1) determining a level of each of the biomarkers of a biomarker composition in a sample from the subject, wherein the biomarker composition comprises N-Acetyl-D-glucosamine 6-phosphate (GlcNAc-6-P) and mannitol, wherein the sample is urine; 2) comparing the level of each of the biomarkers of step 1) with a reference dataset or a reference value.

The present invention also provides a method for screening a candidate compound for treatment of CHD, wherein the method comprises the following steps: 1) determining a level of each of the biomarkers of a biomarker composition in a sample collected from a non-human subject that has been administered with the candidate compound, wherein the biomarker composition comprises N-Acetyl-D-glucosamine 6-phosphate (GlcNAc-6-P) and mannitol, and the sample is urine; 2) comparing the level of each of the biomarkers of step 1) with the level before administering the candidate compound, wherein optionally, the biomarker composition further comprises one or more selected from creatine and phytosphingosine.

The present invention provides a method for evaluation of the effect of a treatment of CHD, wherein the method comprises the following steps: 1) determining a level of each of the biomarkers of a biomarker composition in a sample collected from a subject that has been treated for CHD, wherein the biomarker composition comprises N-Acetyl-D-glucosamine 6-phosphate (GlcNAc-6-P) and mannitol, and the sample is urine; 2) comparing the level of each of the biomarkers of step 1) with the level before the treatment to the subject, wherein optionally, the biomarker composition further comprises one or more selected from creatine and phytosphingosine.

### FURTHER DISCLOSURE

The first aspect of the disclosure relates to a biomarker composition, which comprises one or more selected from the group consisting of: N-Acetyl-D-glucosamine 6-phosphate (GlcNAc-6-P), mannitol, creatine and phytosphingosine.

In an aspect of the disclosure, the biomarker composition comprises GlcNAc-6-P and/or mannitol.

In an aspect of the disclosure, the biomarker composition comprises GlcNAc-6-P and one or more selected from mannitol, creatine and phytosphingosine.

In an aspect of the disclosure, the biomarker composition comprises mannitol and one or more selected from GlcNAc-6-P, creatine and phytosphingosine.

In an aspect of the disclosure, the biomarker composition comprises GlcNAc-6-P and mannitol.

In an aspect of the disclosure, the biomarker composition comprises GlcNAc-6-P and mannitol, and and one or more selected from creatine and phytosphingosine.

In an aspect of the disclosure, N-Acetyl-D-glucosamine 6-phosphate (GlcNAc-6-P), mannitol, creatine and phytosphingosine come from urine.

In an aspect of the disclosure, when compared with healthy controls, the increase of GlcNAc-6-P and/or mannitol indicates that the subject is in the risk of CHD or has CHD.

In an aspect of the disclosure, when compared with healthy controls, the decrease of creatine and/or phytosphingosine indicates that the subject is in the risk of CHD or has CHD.

In an aspect of the disclosure, the step of determining the levels of the biomarkers are carried out by mass spectrometry, preferably, mass spectrometry in conjunction with chromatography, such as gas chromatography mass spectrometry (GC-MS) or liquid chromatography mass spectrometry (LC-MS).

The second aspect of the disclosure relates to a reagent composition, which comprises the reagents used for detection of each of the biomarker composition according to any item of the first aspect of the disclosure.

In an aspect of the disclosure, the reagents comprise substances used in mass spectrometry for detection of the biomarker composition according to any item of the first aspect of the disclosure.

In an aspect of the disclosure, the sample for detection of the biomarkers is urine.

The third aspect of the disclosure relates to a kit, which comprises the biomarker composition according to any item of the first aspect of the disclosure and/or the reagent composition according to any item of the second aspect of the disclosure.

In an aspect of the disclosure, wherein the kit further comprises the training dataset of the levels of the biomarkers in the biomarker composition according to any item of the first aspect of the disclosure for CHD patients and healthy controls (for example as shown by Table 7).

The fourth aspect of the disclosure relates to a use of the biomarker composition according to any item of the first aspect of the disclosure and/or the reagent composition according to any item of the second aspect of the disclosure in the preparation of a kit, wherein the kit is used for evaluation of the risk of CHD in a subject, or for use in diagnosis of CHD in a subject.

In an aspect of the disclosure, the evaluation or diagnosis comprises the following steps: 1) determining the level of each of the biomarkers of the biomarker composition according to claim 1 or 2 in a sample from the subject; 2) comparing the level of step 1) with a reference dataset or a reference value (for example a reference value of healthy controls); preferably, the reference dataset comprises the level of the biomarker of the biomarker composition according to any item of the first aspect of the disclosure in a sample from CHD patients and healthy controls.

In an aspect of the disclosure, the sample is urine.

In an aspect of the disclosure, comparing the level of step 1) with a reference dataset further comprises the step of executing a multivariate statistical model to output the probability of illness; preferably, the multivariate statistical model is random forest model.

In an aspect of the disclosure, the subject is determined as being at risk of CHD or having CHD if the probability of illness ≥0.5.

In an aspect of the disclosure, when compared with a reference value, the increase of GlcNAc-6-P and/or mannitol indicates that the subject is in the risk of CHD or has CHD.

In an aspect of the disclosure, when compared with a reference value, the decrease of creatine and/or phytosphingosine indicates that the subject is in the risk of CHD or has CHD.

In an aspect of the disclosure, the step of determining the level of each of the biomarkers are carried out by mass spectrometry, preferably, mass spectrometry in conjunction with chromatography, such as gas chromatography mass spectrometry (GC-MS) or liquid chromatography mass spectrometry (LC-MS).

In an aspect of the disclosure, the method further comprises the step of processing the sample before step 1).

In an aspect of the disclosure, the kit further comprises the training dataset of the levels of the biomarker composition according to any item of the first aspect of the disclosure for CHD patients and/or healthy controls (for example as shown by Table 7).

The invention provides a method for diagnosis of CHD in a subject, wherein the method comprises the following steps: 1) determining the level of each of the biomarkers of a biomarker composition in a sample from the subject, wherein the biomarker composition comprises N-Acetyl-D-glucosamine 6-phosphate (GlcNAc-6-P) and mannitol, wherein the sample is urine; 2) comparing the level of each of the biomarkers of step 1) with a reference dataset or a reference value. In an embodiment of the invention, the reference value is a reference value of healthy controls. Preferably, the reference dataset comprises the level of each of the biomarkers in samples from CHD patients and healthy controls.

In an embodiment of the invention, step 2) further comprises the step of executing a multivariate statistical model to output the probability of illness; preferably, the multivariate statistical model is random forest model.

In an embodiment of the invention, the subject is determined as being at risk of CHD or having CHD if the probability of illness ≥0.5.

In an embodiment of the invention, when compared with a reference value, an increase of GlcNAc-6-P and/or mannitol indicates that the subject has CHD.

In an embodiment of the invention, when compared with a reference value, a decrease of creatine and/or phytosphingosine indicates that the subject has CHD.

In an embodiment of the invention, the step of determining the level of each of the biomarkers is carried out by mass spectrometry, preferably, mass spectrometry in conjunction with chromatography, such as gas chromatography mass spectrometry (GC-MS) or liquid chromatography mass spectrometry (LC-MS).

In an aspect of the disclosure, the method further includes setting up the training dataset of the levels of the biomarker composition for CHD patients and/or healthy controls (for example as shown by Table 7).

In an embodiment of the invention, the method further comprises the step of processing the sample before step 1).

The sixth aspect of the disclosure relates to the biomarker composition according to any item of the first aspect of the disclosure or the reagent composition according to any item of the second aspect of the disclosure, for use in a method of evaluation of the risk of CHD in a subject or for diagnosis of CHD in a subject.

In an aspect of the disclosure, the method of evaluation or diagnosis comprises the following steps: 1) determining the level of each of the biomarkers of the biomarker composition according to claim 1 or 2 in a sample from the subject; 2) comparing the level of step 1) with a reference dataset or a reference value (for example a reference value of healthy controls); preferably, the reference dataset comprises the level of the biomarker of the biomarker composition according to any item of the first aspect of the disclosure in a sample from CHD patients and healthy controls.

In an aspect of the disclosure, the sample is urine.

In an aspect of the disclosure, comparing the level of step 1) with a reference dataset further comprises the step of executing a multivariate statistical model to output the probability of illness; preferably, the multivariate statistical model is random forest model.

In an aspect of the disclosure, the subject is determined as being at risk of CHD or having CHD if the probability of illness ≥0.5.

In an aspect of the disclosure, when compared with a reference value, the increase of GlcNAc-6-P and/or mannitol indicates that the subject is in the risk of CHD or has CHD.

In an aspect of the disclosure, when compared with a reference value, the decrease of creatine and/or phytosphingosine indicates that the subject is in the risk of CHD or has CHD.

In an aspect of the disclosure, the step of determining the level of each of the biomarkers are carried out by mass spectrometry, preferably, mass spectrometry in conjunction with chromatography, such as gas chromatography mass spectrometry (GC-MS) or liquid chromatography mass spectrometry (LC-MS).

In an aspect of the disclosure, the method further includes setting up the training dataset of the levels of the biomarker composition according to claim 1 or 2 for CHD patients and/or healthy controls (for example as shown by Table 7).

In an aspect of the disclosure, the method further comprises the step of processing the sample before step 1).

The seventh aspect of the disclosure relates to a use of the biomarker composition according to any item of the first aspect of the disclosure and/or the reagent composition according to any item of the second aspect of the disclosure in the preparation of a kit, wherein the kit is used for screening candidate compounds for treatment of CHD in a subject, or for evaluating the effect of the treatment of CHD in a subject.

In an aspect of the disclosure, the method of screening or evaluation comprises the following steps: 1) determining the level of each of the biomarkers of the biomarker composition according to any item of the first aspect of the disclosure in a sample from the subject after administering the candidate compounds or the treatment to the subject; 2) comparing the level of step 1) with the level of the above mentioned biomarker before administering the candidate compounds or the treatment to the subject.

In an aspect of the disclosure, the sample is urine.

In an aspect of the disclosure, the decrease of GlcNAc-6-P and/or mannitol indicates that the compound is a candidate compound for treatment of CHD in a subject or the treatment of CHD in the subject is effective.

In an aspect of the disclosure, the increase of creatine and/or phytosphingosine indicates that the compound is a candidate compound for treatment of CHD in a subject or the treatment of CHD in the subject is effective.

In an aspect of the disclosure, the step of determining the level of each of the biomarkers are carried out by mass spectrometry, preferably, mass spectrometry in conjunction with chromatography, such as gas chromatography mass spectrometry (GC-MS) or liquid chromatography mass spectrometry (LC-MS).

In an aspect of the disclosure, the method further comprises the step of processing the sample before step 1).

The invention further provides a method for screening a candidate compound for treatment of CHD, wherein the method comprises the following steps: 1) determining the level of each of the biomarkers of the biomarker composition in a sample collected from the non-human subject that has been administered with the candidate compound, wherein the biomarker composition comprises N-Acetyl-D-glucosamine 6-phosphate (GlcNAc-6-P) and mannitol, and the sample is urine; 2) comparing the level of each of the biomarkers of step 1) with the level before administering the candidate compound, wherein optionally, the biomarker composition further comprises one or more selected from creatine and phytosphingosine.

In an embodiment of the invention, the decrease of GlcNAc-6-P and/or mannitol indicates that the compound is a candidate compound for treatment of CHD in a subject or the treatment of CHD in the subject is effective.

In an embodiment of the invention, the increase of creatine and/or phytosphingosine indicates that the compound is a candidate compound for treatment of CHD in a subject or the treatment of CHD in the subject is effective.

In an embodiment of the invention, the step of determining the level of each of the biomarkers are carried out by mass spectrometry, preferably, mass spectrometry in conjunction with chromatography, such as gas chromatography mass spectrometry (GC-MS) or liquid chromatography mass spectrometry (LC-MS).

In an embodiment of the invention, the method further comprises the step of processing the sample before step 1).

The ninth aspect of the disclosure relates to the biomarker composition according to any item of the first aspect of the disclosure, for use in a method of screening candidate compounds for treatment of CHD in a subject or for evaluation of the effect of the treatment of CHD in a subject.

In an aspect of the disclosure, the method of screening or evaluation comprises the following steps: 1) determining the level of each of the biomarkers of the biomarker composition according to any item of the first aspect of the disclosure in a sample from the subject after administering the candidate compounds or the treatment to the subject; 2) comparing the level of step 1) with the level of the above mentioned biomarker before administering the candidate compounds or the treatment to the subject.

In an aspect of the disclosure, the sample is urine.

In an aspect of the disclosure, the decrease of GlcNAc-6-P and/or mannitol indicates that the compound is a candidate compound for treatment of CHD in a subject or the treatment of CHD in the subject is effective.

In an aspect of the disclosure, the increase of creatine and/or phytosphingosine indicates that the compound is a candidate compound for treatment of CHD in a subject or the treatment of CHD in the subject is effective.

In an aspect of the disclosure, the step of determining the level of each of the biomarkers are carried out by mass spectrometry, preferably, mass spectrometry in conjunction with chromatography, such as gas chromatography mass spectrometry (GC-MS) or liquid chromatography mass spectrometry (LC-MS).

In an aspect of the disclosure, the method further comprises the step of processing the sample before step 1).

The invention also provides a method for setting up a mass spectrometry model for diagnosis of CHD in a subject, which comprises the step of identifying the differentially expressed substance in urine between CHD patients and healthy controls, wherein the differentially expressed substance comprises one or more selected from the group consisting of: N-Acetyl-D-glucosamine 6-phosphate (GlcNAc-6-P), mannitol, creatine and phytosphingosine.

The eleventh aspect of the disclosure relates to a biomarker composition, which comprises one or more selected from the group consisting of: *Streptococcus sp. M334, Streptococcus sp. M143* and *Clostridium sp. HGF2.*

In an aspect of the disclosure, the biomarker composition comprises *Clostridium sp. HGF2.*

In an aspect of the disclosure, the biomarker composition comprises *Streptococcus sp. M334, Streptococcus sp. M143* and *Clostridium sp. HGF2.*

In an aspect of the disclosure, when compared with healthy controls, the increase of the levels of the biomarkers of the biomarker composition indicates that the subject is in the risk of CHD or has CHD.

The twelfth aspect of the disclosure relates to a reagent composition, which comprises the reagents used for detection of the biomarker composition according to any item of the eleventh aspect of the disclosure.

The present disclosure further relates to a kit, which comprises the biomarker composition according to any item of the eleventh aspect of the disclosure and/or the reagent composition according to any item of the twelfth aspect of the disclosure.

The present disclosure further relates to a use of the biomarker composition according to any item of the eleventh aspect of the disclosure and/or the reagent composition according to any item of the twelfth aspect of the disclosure in the preparation of a kit, wherein the kit is used for evaluation of the risk of CHD in a subject, or for use in diagnosis of CHD in a subj ect.

In an aspect of the disclosure, the evaluation or diagnosis comprises the following steps:1) determining the level of each of the biomarkers of the biomarker composition according to any item of the eleventh aspect of the disclosure in a fecal sample from the subject; 2) comparing the level of step 1) with a reference value ( for example a reference value of healthy controls).

In an aspect of the disclosure, when compared with a reference value, the increase of the levels of the biomarkers of the biomarker composition according to any item of the eleventh aspect of the disclosure indicates that the subject is in the risk of CHD or has CHD.

The present disclosure further relates to a method for evaluation of the risk of CHD in a subject or for diagnosis of CHD in a subject, wherein the method comprises the following steps:1) determining the level of each of the biomarkers of the biomarker composition according to any item of the eleventh aspect of the disclosure in a fecal sample from the subject; 2) comparing the level of step 1) with a reference value ( for example a reference value of healthy controls).

In an aspect of the disclosure, when compared with a reference value, the increase of the levels of the biomarkers of the biomarker composition according to any item of the eleventh aspect of the disclosure indicates that the subject is in the risk of CHD or has CHD.

The present disclosure further relates to the biomarker composition according to any item of the eleventh aspect of the disclosure, for use in a method of evaluation of the risk of CHD in a subject or for diagnosis of CHD in a subject.

In an aspect of the disclosure, the method of evaluation or diagnosis comprises the following steps: 1) determining the level of each of the biomarkers of the biomarker composition according to any item of the eleventh aspect of the disclosure in a fecal sample from the subject; 2) comparing the level of step 1) with a reference value (for example a reference value of healthy controls).

In an aspect of the disclosure, when compared with a reference value, the increase of the levels of the biomarkers of the biomarker composition according to any item of the eleventh aspect of the disclosure indicates that the subject is in the risk of CHD or has CHD.

The present disclosure further relates to a use of the biomarker composition according to any item of the eleventh aspect of the disclosure and/or the reagent composition according to any item of the twelfth aspect of the disclosure in the preparation of a kit, wherein the kit is used for screening a candidate compound for treatment of CHD in a subject, or for evaluation the effect of the treatment of CHD in a subject.

In an aspect of the disclosure, the method of screening or evaluation comprises the following steps: 1) determining the level of each of the biomarkers of the biomarker composition according to claim 53 or 54 in a sample from the subject after administering the candidate compounds or the treatment to the subject; 2) comparing the level of step 1) with the level of the above mentioned biomarker before administering the candidate compounds or the treatment to the subject.

In an aspect of the disclosure, the decrease of the levels of the biomarkers of the biomarker composition according to any item of the eleventh aspect of the disclosure indicates that the compound is a candidate compound for treatment of CHD in a subject or the treatment of CHD in the subject is effective.

The present invention further provides a method for evaluation of the effect of a treatment of CHD, wherein the method comprises the following steps: 1) determining the level of each of the biomarkers of the biomarker composition in a sample collected from a subject that has been treated for CHD, wherein the biomarker composition comprises N-Acetyl-D-glucosamine 6-phosphate (GlcNAc-6-P) and mannitol, and the sample is urine; 2) comparing the level of each of the biomarkers of step 1) with the level before treatment to the subject, wherein optionally, the biomarker composition further comprises one or more selected from creatine and phytosphingosine.

In an embodiment of the invention, the decrease of GlcNAc-6-P and/or mannitol indicates that the compound is a candidate compound for treatment of CHD in a subject or the treatment of CHD in the subject is effective.

The present disclosure further relates to the biomarker composition according to any item of the eleventh aspect of the disclosure, for use in a method of screening candidate compounds for treatment of CHD in a subject or for evaluation of the effect of the treatment of CHD in a subject.

In an aspect of the disclosure, the method of screening and evaluation comprises the following steps: 1) determining the level of each of the biomarkers of the biomarker composition according to claim 53 or 54 in a sample from the subject after administering the candidate compounds or the treatment to the subject; 2) comparing the level of step 1) with the level of the above mentioned biomarker before administering the candidate compounds or the treatment to the subject.

In an aspect of the disclosure, the decrease of the levels of the biomarkers of the biomarker composition according to any item of the eleventh aspect of the disclosure indicates that the compound is a candidate compound for treatment of CHD in a subject or the treatment of CHD in the subject is effective.

Terms used herein have meanings as commonly understood by a person of ordinary skill in the fields to which the present invention is relevant. However, in order to better understand the invention, the definitions and explanations of the relevant terms are provided as follows.

The term "coronary heart disease (CHD)", also known as "coronary artery disease (CAD)", is a group of diseases that includes: stable angina, unstable angina, myocardial infarction, and sudden coronary death, etc. Patients with CHD are diagnosed by coronary angiography techniques.

Mass spectrometry (MS) can be classified into ion traps, quadrupole, orbitrap and time-of-flight mass spectrometry, and the deviations are 0.2amu, 0.4amu, 3ppm, 5ppm, respectively. The MS data is acquired with orbitrap mass spectrometry.

The levels of the biomarkers are indicated by peak intensity in MS.

Mass-to-charge (m/z) and retention time (RT) have the same meaning known by prior art. The unit of m/z is amu, which refers to atomic mass unit, also named as Dalton. Dalton is the standard unit that is used for indicating mass on an atomic or molecular scale (atomic mass). One unified atomic mass unit is equivalent to 1 g/mol. It is defined as one twelfth of the mass of an unbound neutral atom of carbon-12 in its nuclear and electronic ground state.

The person skilled in the art knows that the values of m/z and retention time will vary within a certain range when different detection methods or LC-MS instruments are used. For example, m/z can vary in the range of ±3.00, or ±2.00, or ± 1.00, and retention time can vary in the range of ±60s, or ±45s, or ±30s, or ± 15s.

In an aspect of the disclosure, a reference dataset refers to a training dataset.

Training datasets and validation datasets have the same meaning known by prior art. The training datasets refer to a collection of data of the levels of biomarkers in biological samples for CHD patients and healthy controls. The validation datasets refer to a collection of data used to test the performance of the training datasets. The levels of biomarkers can be indicated as absolute values or relative values according to the method of determination. For example, when mass spectrometry is used to determine the levels of the biomarkers, the intensity of a peak can represent the levels of the biomarkers, which is a relative value; when PCR is used to determine the levels of the biomarkers, the copy numbers of a gene or the copy numbers of fragments of a gene can represent the levels of the biomarkers.

In an embodiment of the invention, a reference value refers to a reference value of healthy controls or a normal value. The person skilled in the art knows that range of normal value (absolute value) of each biomarker in sample can be obtained with test and calculation method well known in the art when sample size is large enough. Thus, when other methods except mass spectrometry are applied to detect the levels of biomarkers, the absolute values of the levels of biomarkers in samples can be directly compared with normal values, in order to evaluate the risk of CHD and diagnosis or early diagnosis of CHD, optionally, statistical methods can be included.

The term "a biomarker", also named "a biological marker", refers to a measurable indicator of a biological state or condition of a subject. Such biomarkers can be any substance in the subject, for example, nucleic acid marker (e.g. DNA), protein marker, cytokine marker, chemokine marker, carbohydrate marker, antigen marker, antibody marker, species marker (species/genus marker) and functions marker (KO/OG marker) and the like, provided that they are in relation with a particular biological state or condition (such as, a disease) of the subject. Biomarkers are often measured and evaluated to examine normal biological processes, pathogenic processes, or pharmacologic responses to a therapeutic intervention, and are useful in many scientific fields.

The term "a biomarker set" refers to a set of biomarkers (that is, a combination of two or more biomarkers).

The term "a subject" refers to an animal, particularly a mammal, such as a primate, preferably human.

The term "plasma" refers to the fluid component of the whole blood. Depending on the separation method used, plasma may be completely free of cellular components, or may contain various amounts of platelets and/or small amounts of other cellular components.

The low-molecular-weight metabolites ( < 1500Da) in the urine samples are extracted for HPLC-MS experiments. The extraction method is well known in the art. Organic solvent is used to precipitate protein, the obtained mixture is centrifuged, and then supernatant is transferred for metabolic profiling by HPLC-MS.

Terms such as "a", "an" and "the" are not intended to refer to only a singular entity, but include the general class of which a specific example may be used for illustration.

It would be appreciated by those skilled in the art that the terminology herein is provided for better understanding of the present invention, but is not intended to delimit the invention, except as outlined in the claims.

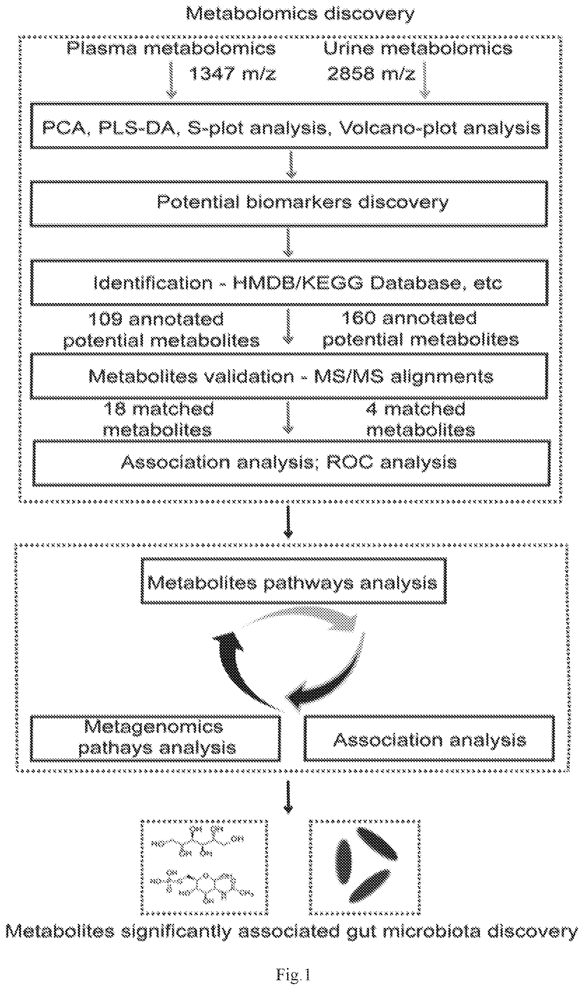

The present invention is based on the following findings by the inventors:
Non-invasive and highly accurate approaches to diagnose and predict CHD are urgently needed. To explore potential characteristic metabolites signatures associated with CHD, non-targeted metabolomics technique is performed to analyze plasma, urine samples and metagenomics technology is applied to further validate the metabolites with potential origin from the fecal metagenomics data of CHD patients and healthy subjects. The workflow is shown in Fig. 1. Statistical and bioinformatics methods are used to identify significantly different mass-to-charge (m/z) that can discriminate CHD cases from healthy controls. Hierarchical cluster analysis (HCA) is performed to identify m/z clusters contributing to phenotype separation and spearman correlation analysis is applied to identify potential biomarkers' correlations related to abnormal functions. The identified significantly changed metabolites are validated using purchased standards. Several significantly different metabolites are correlated with intestine flora on ECs, KOs and species levels. This study proves the power of potential noninvasive biomarkers discovery in biofluids of patients and the integrated analysis of metabolomics and metagenomics would pave the way to reveal the interactions between host and gut microbes.

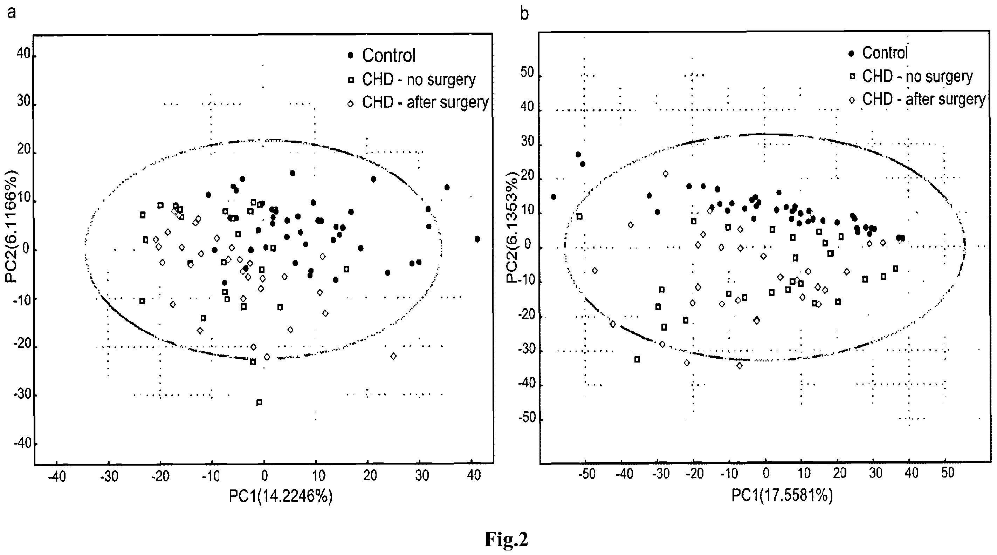

Plasma and urine samples from CHD patients and control healthy people were analyzed using untargeted metabolomics technique. In plasma, 18 significantly changed metabolites (13 LPCs, 2 glycerophosphocholines, L-Arginine, GlcNAc-6-P and paraxanthine) were identified as potential biomarkers. In urine, 4 significantly changed metabolites (GlcNAc-6-P, mannitol, creatine, phytosphingosine) were identified as potential biomarkers in CHD patients. To access the clinical relevance of these potential biomarkers, the diagnostic capability of these 22 metabolites were evaluated by ROC. GlcNAc-6-P appears the most discriminative biomarker which shows relatively good diagnostic ability with FN of 0.153 and FP of 0.208. Correlation analysis between potential biomarkers and biochemical clinical data suggest plasma LPCs are significantly positive correlated with cholesterol (CHOL), high-density lipoprotein (HDLC), and total protein (TP), while GlcNAc-6-P and L-arginine exhibit negative correlation with CHOL, HDLC, and TP. This suggests the metabolites may potentially influence the normal metabolic pathways in our body. Among these 59 CHD patients, 32 patients had undergone Percutaneous Coronary Intervention (PCI) before but no difference had been observed between these 32 postoperative patients group and those 27 patients group with no surgery (as shown in PCA score plots in Fig. 2), suggesting the PCI did not influence the whole metabolic pattern in patients.

As estimated, over 30% of metabolites in human body originate from intestinal microbes and may contribute to host diseases. In this study, metabolomics and metagenomics techniques were integrated and evidence that microbial species and their associated metabolites were involved in CHD diseases were uncovered for the first time. Firstly, mannitol was identified as a potential urine biomarker in CHD patients. The fact that no related homo sapiens enzymes are found in the fructose and mannose metabolism so far indicates mannitol should belong to microbial metabolites family. Mannitol was previously reported to be produced by lactic acid bacteria and pseudomonas putida. In the current study, spearman correlation analysis of, KOs, species and mannitol indicates that three gut flora species, *Clostridium sp. HGF2, Streptococcus sp. M334,* and *Streptococcus sp. M143,* play important roles in metabolism of mannitol. This was further validated by mannose-specific IIB component of PTS system (EC:2.7.1.69) which was found to be the common enzyme in all three CHD enriched gut microbiota species. Secondly, GlcNAc-6-P, an endogenous and microbial metabolite, was identified in both plasma and urine samples of CHD patients. GlcNAc-6-P participates in sugar metabolism with dual functions in regulating host cardiovascular activity. Previous literature shows that *Escherichia coli* could metabolize anhydro-N-acetylmuramic acid obtained either from the environment or its own cell wall to N-acetylglucosamine-phosphate (Uehara, T., Suefuji, K., Jaeger, T., Mayer, C. &Park, J.T. Mur Q etherase is required by Escherichia coli in order to metabolize anhydro-N-acetylmuramic acid obtained either from the environment or from its own cell wall. J. Bacteriol. 188, 1660-1662 (2006)), which would then be converted to GlcNAc-6-P in amino sugar and nucleotide sugar metabolism. In our study, the metabolism of GlcNAc-6-P was found to be significantly correlated with *Clostridium sp. HGF2* by NagA (EC:3.5.1.25) and N-acylglucosamine-6-phosphate 2-epimerase (EC:5.1.3.9). GlcNAc-6-P can be converted into glucosamine-6-P by NagA (EC:3.5.1.25) enzyme which plays a central role in microbial cell wall synthesis and glycolysis.

The discovery of these two microbial metabolites (Mannitol and GlcNAc-6-P) and their correlated microbiota in CHD patients has two important implications. First, it confirmed that microbial metabolites can be used as potential biomarkers for CHD diagnosis along with other traditional metabolites. For instance, GlcNAc-6-P in urine exhibited relatively strong CHD diagnostic ability with AUC of 0.88 and showed FN of 0.153 and FP of 0.208 in the ROC analysis of validation dataset. Second, microbial metabolites reflect the abnormalities of the host intestine microbiota, so new strategy for CHD treatments can be developed by adjusting patients gut intestine ecosystem. In the future, microbial species and their associated metabolites could be used as new indexes and targets for diagnosis and treatment of CHD.

In summary, non-targeted metabolomics technology and metagenomics technology were integrated for CHD study in this study. This combinational work not only leads to important biological candidate biomarkers discovery in CHD, but also bridges the gap between our human systems and intestine microbiota and provides novel insights into the microbial species related to CHD.

The present disclosure is further exemplified in the following non-limiting Examples. Unless otherwise stated, parts and percentages are by weight and degrees are Celsius. The agents as used were all commercially available. As apparent to one of ordinary skill in the art, these Examples, while indicating preferred embodiments of the invention, are given by way of illustration only.

### BRIEF DISCRIPTION OF DRAWINGS

These and other aspects and advantages of the present disclosure will become apparent and more readily appreciated from the following descriptions taken in conjunction with the drawings, in which:
**Fig. 1** | **The workflow in our study.** Firstly, potential biomarkers discovery in plasma and urine had been performed. Secondly, pathway analysis and association analysis of potential biomarkers and gut flora had been applied. Lastly, potential biomarkers associated gut flora species had been discovered.
**Fig. 2** **| PCA Scores plot of 32 postoperative patients and 27 no operative patients with 43 healthy controls. (a)** The plasma samples of postoperative CHD patients and no operative CHD patients gathered together showing no significant difference among them. **(b)** The scattered distribution of urine samples in postoperative CHD patients and no operative CHD patients also suggested no significant difference among them.

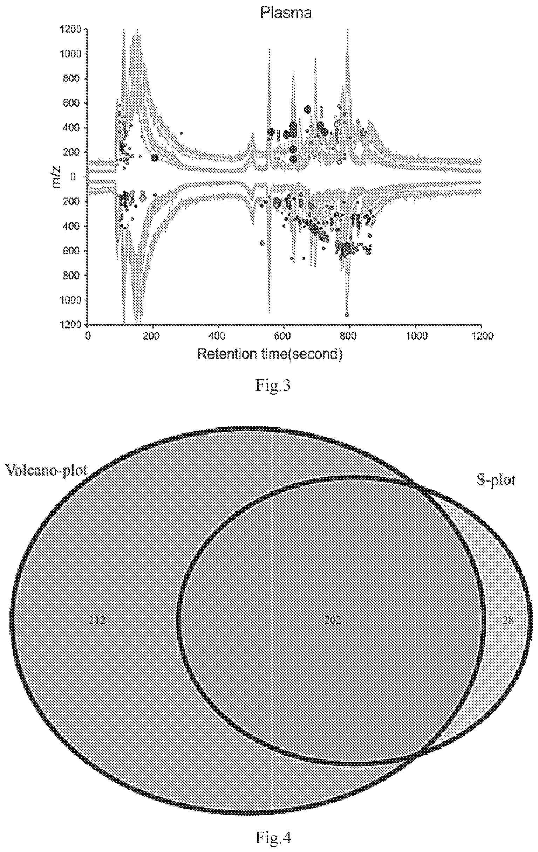

**Fig. 3** **| Potential biomarkers discovery in plasma metabolomics.** Cloud plot of plasma metabolites profiles. Upper and under circles indicated metabolites with increased *(fold change >* 1.2, 196 metabolites) and decreased intensity *(fold change* < 0.8, 319 metabolites) in CHD patients' plasma samples compared with healthy controls. The darkness of color is correlated with adjusted *p.value* (named as *q.value):* color from white to dark black indicated smaller adjusted *p. value.* The area of circle is correlated with magnitude of intensity change: In the upper part, the bigger the circle was, the more enriched metabolites were in CHD patients' plasma samples compared with healthy controls' plasma samples. While in the under part, the bigger the circle was, the more enriched metabolites were in healthy controls'.
**Fig. 4** | **Plasma metabolomics statistical analysis.** In Veen-plot, Volcano-plot and S-plot were integrated and there were 202 overlapped m/z that were significantly different between CHD patients' plasma samples and healthy controls' plasma samples.
**Fig. 5** | **Boxplot of 15 choline metabolites.**

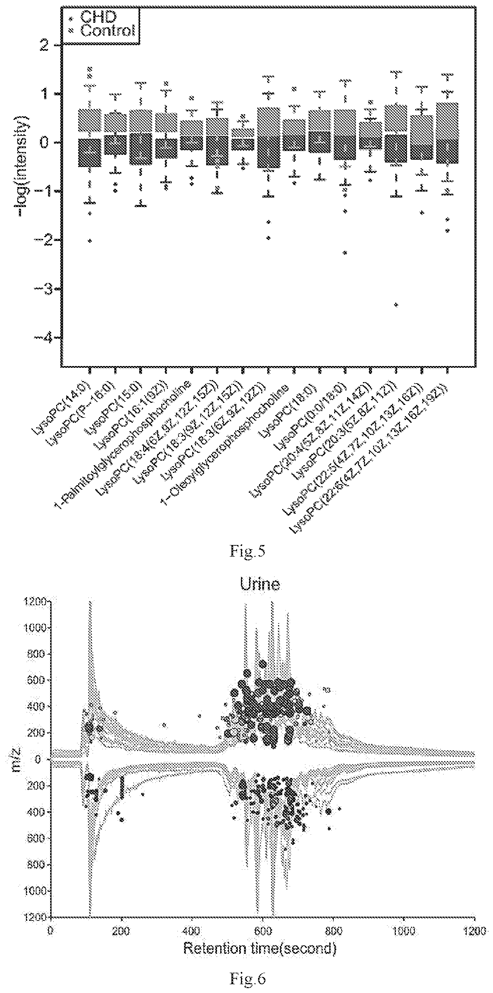

**Fig. 6** | **Cloud plot of urine metabolites profiles.** Upper and under circles indicated metabolites with increased *(fold change >* 1.2, 196 metabolites) and decreased intensity *(fold change <* 0.8, 319 metabolites) in CHD patients' urine samples compared with healthy controls. The darkness of color is correlated with adjusted *p.value* (named as *q.value):* color from white to dark black indicated smaller adjusted *p. value.* The area of circle is correlated with magnitude of intensity change: In the upper part, the bigger the circle was, the more enriched metabolites were in CHD patients' urine samples compared with healthy controls' urine samples. While in the under part, the bigger the circle was, the more enriched metabolites were in healthy controls.
**Fig. 7** **| Urine metabolomics statistical analysis.** In Veen-plot, the overlapped 391 m/z were significantly different metabolites between CHD urine samples and control urine samples by the integration of Volcano-plot and S-plot analysis.
**Fig. 8** **| Veen diagram of all significant differential metabolites in plasma and urine.**
**Fig. 9** **| Receiver operating characteristic (ROC) analysis of potential biomarkers.** ROC analysis and boxplots of 7 identified plasma potential biomarkers (a-g) and 2 identified urine potential biomarkers (h-i) with good diagnostic capability among 176 additional plasma samples (98 controls vs 78 CHD patients) and 395 additional urine samples (173 controls vs 222 CHD patients) respectively.
Fig.10 | ROC analysis of 7 identified plasma potential biomarkers with good diagnostic capability among plasma validation datasets (78 CHD patients plasma samples VS 98 healthy controls plasma samples).
Fig. 11 | ROC analysis of 2 identified urine potential biomarkers with good diagnostic capability among urine validation datasets (222 CHD patients urine samples VS 173 healthy controls urine samples).
**Fig. 12** **| A workflow for the discovery of interactions between metabolites and gut microbiota.** Pathways analysis and association analysis among plasma, urine potential biomarkers and gut microbiota were implemented. *Clostridium sp. HGF2* was found to significantly correlate with GlcNAc-6-P. *Clostridium sp. HGF2, Streptococcus sp. M143, Streptococcus sp. M334* were found to significantly associate with mannitol.
Fig.13 | ROC analysis of *Clostridium sp. HGF2* biomarkers with good diagnostic capability among new fecal samples (59 CHD patients fecal samples VS 43 healthy individuals fecal samples).

### EXAMPLES

### Example 1. Identifying and validating biomarkers for evaluating risk of CHD related diseases

**1.1 Materials.** Formic acid and methanol (HPLC grade) were purchased from Fisher Scientific Corporation (Loughborough, UK). Water used in the experiments was obtained from a Milli-Q Ultra-pure water system (Millipore, Billerica, MA). An Agilent ZORBAX ODS C-18 column (150 mm×2.1 mm, 3.5 µm, Agilent, USA) was used for all analysis.

**1.2 Clinical samples.** All patients with CHD diagnosed by coronary angiography techniques were recruited from the Guangdong General Hospital. All control people enrolled in our study were free of clinically evident CHD at medical examination during the same period.

Paired plasma, urine and fecal samples of CHD patients (n=59) and healthy controls (n=43) were obtained from the Guangdong General Hospital. Coronary angiography techniques were performed to diagnose CHD patients recruited in this study. The healthy control had underwent physical examination in the same hospital. Patients and controls did not receive probiotics or antibiotics within one month before sample collection. Among these 59 CHD patients, 32 patients had undergone Percutaneous Coronary Intervention (PCI) before. The participants' clinical information was provided in Supplementary Table 1. Besides, 176 additional plasma samples (98 controls vs 78 CHD patients) and 395 additional urine samples (173 controls vs 222 CHD patients) were included for potential biomarkers diagnostic capability analysis. Venous blood and midstream urine were collected in the morning before breakfast from all participants. Venous blood collected by Vacuette EDTA blood collection tubes were centrifuged at 2200x g for 5 min at 4 °C to obtain plasma samples. The plasma and urine samples were stored at -80 °C until use.

The corresponding fresh stool samples were collected from CHD patients (n=59) and healthy controls (n=43) on the same day at hospital. Samples were mechanically homogenized with a sterile spatula, and then aliquots containing 1g of stool in a 12ml sterile cryovial were made using the Sarstedt stool sampling system (Sarstedt, Nümbrecht, Germany). The aliquots were then stored in freezers at -20 °C and transported to the laboratory with ice pack within 48 h after collection. After that, fecal samples were stored at -80 °C until use. These protocols were reviewed by the Institutional Review Board of BGI-Shenzhen. Before collecting samples, patients were informed and written consent were obtained from them.

**Samples preparations for HPLC-MS experiments.** To extract the low-molecular-weight metabolites (<1500Da) in the plasma and urine samples, some modifications were made to the procedures reported previously (Luan, H. et al. Serum metabolomics reveals lipid metabolism variation between coronary artery disease and congestive heart failure: a pilot study. Biomarkers.18, 314-321 (2013)). Before experiments, all plasma and urine samples were thawed on ice and a "quality control" (QC) sample was made by mixing and blending equal volumes (10µL) from each plasma or urine samples which was applied to estimate a "mean" profile representing all the analytes encountered during analysis. Then low molecular weight metabolites (<1500Da) were extracted from the plasma and urine samples using the following procedure: Plasma samples were mixed with methanol (1:2 v/v) while urine samples were mixed with methanol (1:1 v/v) to precipitate protein. The plasma and urine sample mixture were centrifuged at 14000x g for 10 min at 4°C and then supernatant was transferred into a 1.5 mL polypropylene tube for metabolic profiling by HPLC-MS.

**HPLC-MS experiments.** Shimadzu Prominence HPLC system (Shimadzu) was coupled to a LTQ Orbitrap Velos instrument (Thermo Fisher Scientific, MA, USA) set at 30000 resolution to acquire HPLC-MS data. An Agilent ZORBAX ODS C18 column (150 mm×2.1 mm, 3.5 µm, Agilent, USA) was used. Sample analysis was performed in positive ion modes with a spray voltage of 4.5 kV and capillary temperature of 350°C. The mass scanning range was 50-1500 m/z. The flow rates of nitrogen sheath gas and nitrogen auxiliary gas were set to 30 L/min and 10 L/min, respectively. The HPLC-MS system was run in binary gradient mode. Solvent A was 0.1% (v/v) formic acid/water, and solvent B was 0.1% (v/v) formic acid/methanol. The gradient was as follows: 5% B at 0 min, 5% B at 5 min, 100% B at 8 min, 100% B at 9 min, 5% B at 18 min, and 5% B at 20 min. The flow rate was 0.2 mL/min. To ensure system equilibrium, the pooled QC sample was injected five times at the beginning. QC sample was injected every five samples during samples detection to further monitor the system stability.

**HPLC-MS data analysis.** The acquired MS data pretreatments including peak picking, peak grouping, retention time correction, second peak grouping, and annotation of isotopes and adducts was performed using the same method as our previously published work (Luan, H. et al. Serum metabolomics reveals lipid metabolism variation between coronary artery disease and congestive heart failure: a pilot study. Biomarkers.18, 314-321 (2013)). LC-MS raw data files were converted into *mzXML* format and then processed by the XCMS and CAMERA toolbox implemented with the R software(v3.1.1). Each ion was identified by combining retention time (RT) and m/z data. Intensities of each peaks were recorded and a three dimensional matrix containing arbitrarily assigned peak indices (retention time-m/z pairs), sample names *(observations)* and ion intensity information *(variables)* was generated.

The obtained matrix was further reduced by removing peaks with more than 80% missing values *(ion intensity* = 0) and those with isotope ions from each groups in order to obtain consistent results. As a quality assurance strategy in metabolic profiling, all retained peaks were normalized to the QC sample using Robust Loess Signal Correction (R-LSC) based on the periodic analysis of a standard biological quality control sample (QC sample) together with the real plasma and urine samples to ensure that the data are of high quality within an analytical run (Dunn, W. B. et al. Procedures for large-scale metabolic profiling of serum and plasma using gas chromatography and liquid chromatography coupled to mass spectrometry. Nat.Protoc.6, 1060-1083 (2011)). The relative standard deviation *(RSD)* values of metabolites in the QC samples was set at a threshold of 30% which was accepted as a standard in the assessment of repeatability in metabolomics data sets.

The nonparametric univariate method (Mann-Whitney-Wilcoxon test) was performed to measure and discover the significantly changed metabolites among the CHD patients and control subjects and then corrected by false discovery rate (FDR) to ensure that metabolite peaks were reproducibly detected. And multivariate statistical analysis (PCA, PLS-DA) were performed to discriminate CHD samples from control subjects. A number of metabolites responsible for the difference in the metabolic profile scan of CHD patients and control subjects can be obtained on the basis of variable importance in the projection (*VIP*) threshold of 1 from the 7-fold cross-validated PLS-DA model. The PLS-DA model was validated at a univariate level using FDR test from the R statistical toolbox with the critical *p. value* set to not higher than 0.05. Permutation multivariate analysis of variance (PERMANOVA), a permutation-based version of the multivariate analysis of variance, was performed in R using the "vegan" package to test the statistical significant differences between metabolic profiles and individuals' phenotypes (Anderson, M. J. A new method for non-parametric multivariate analysis of variance. Aust. Ecol. 26, 32-46 (2001)). Three dimensional PLS-DA analysis was also implemented to show the difference between CHD samples and control subjects. Phenotype analysis was performed to cluster those significantly distributed metabolites. Spearman correlation analysis was implemented among those significantly changed plasma metabolites, urine metabolites and clinical data of CHD patients and control subjects and correlations of metabolites was profiled with Cytoscape software 3.0.2. In addition, receiver operating characteristic (ROC) analysis was used to evaluate diagnostic capability of identified potential biomarkers with the online tool - ROCCET (*http:*//*www.roccet.ca*) (Xia, J.G., Broadhurst, D.I., Wilson, M. & Wishart, D.S. Translational Biomarker Discovery in Clinical Metabolomics: An Introductory Tutorial . Metabolomics 9, 280-299(2012)).

**Metabolites identification and validation.** The online HMDB database (*http:*//*www.hmdb.ca*) and KEGG database (*www.genome.jp*/*kegg*/) were used to identify the metabolites by matching the exact molecular mass data (m/z) of samples with those from database. If a mass difference between observed and the database value was less than 10 ppm, the metabolite would be identified and the molecular formula of metabolites would further be validated by the isotopic distribution measurements. Reference standards were purchased and used to validate and confirm those significantly changed metabolites by comparing their MS/MS spectra and retention time.

### Metabolic profiles of plasma and urine samples

Untargeted metabolomics analysis was performed for the plasma and urine samples from 59 CHD patients and 43 healthy controls. The participants' clinical information was listed in Supplementary Table 1. The detailed workflow was illustrated in Fig. 1. A total of 1347 m/z (mass-to-charge ratio, 93.67%) and 2858 m/z (96.68%) were obtained in plasma and urine samples respectively. The stability and reproducibility of current data was evaluated by the QC samples measured during the whole experimental period. Principle component analysis (PCA) scores plot representation of QC samples for plasma and urine samples were obtained respectively. No drift in the metabolites profiles obtained in positive ion modes, were observed demonstrating good stability and reproducibility in our current metabolomics data set.

### Results for Plasma Samples

For plasma samples, cloud plot analysis of the total 1347 m/z (Fig. 3) showed that the intensity of 196 m/z (14.55%) were increased in CHD patients' plasma samples *(fold change >* 1.2) and 23.68% of them (319 m/z) were decreased in CHD patients' *(fold change <* 0.8). Both PCA scores plot and three-dimensional partial least squares - discriminant analysis (PLS-DA) (Triba, M.N.et al. PLS/OPLS models in metabolomics: impact of permutation of dataset rows on the K-fold cross-validation quality parameters. Mol. Biosyst. 11, 13-19(2015)) scores plot of these plasma samples showed that there were significant differences between 59 CHD patient samples and 43 healthy control samples. CHD patients' plasma samples were apart from healthy control's samples with *PC1, PC2, PC3 as 15.32%, 10.62%, 13.73%* respectively. The permutation multivariate analysis of variance (PERMANOVA) was implemented to test the relation of individual's phenotypes with their metabolite characteristics, and we found CHD status had significant impacts on the metabolic profiling *(P < 0.001,* 1000 permutations) in positive ion mode.

S-plot analysis was used for selection of potentially interesting metabolites biomarkers (Miao, H. et al. Urinary Metabolomics on the biochemical profiles in diet-induced hyperlipidemia rat using ultra-performance liquid chromatography coupled with quadrupole time-of flight SYNAPT high-definition mass spectrometry. J. Anal. Methods.Chem.2014, ID184162 (2014)). Using the criteria that variable importance in the projection (*VIP*) was larger than 1, 230 variables were selected in S-plot. On the condition that *adjusted p. value <* 0.05,*fold change >* 1.2 or < 0.8, 414 variables were retained in Volcano-plot. Combing these two results, 202 shared m/z were obtained (Fig.4). And a total of 109 potential metabolites (20 increased and 89 decreased in CHD patients) were identified by aligning the exactly significant peaks' molecular mass data (m/z) with online database: HMDB and KEGG.

To further identify potential metabolites from 109 m/z, both HMDB and HMDB SERUM databases were searched using accurate mass and mass spectrometric fragmentation patterns (Chen, J. et al. Practical approach for the identification and isomer elucidation of biomarkers detected in a metabonomic study for the discovery of individuals at risk for diabetes by integrating the chromatographic and mass spectrometric information. Anal. Chem. 80, 1280-1289 (2008)). We found 18 matched metabolites from the above database, including 13 Lysophosphatidylcholine (LPCs), 2 glycerophosphocholines, L-Arginine, N-Acetyl-D-glucosamine 6-phosphate (GlcNAc-6-P) and paraxanthine (as listed in Table 1).

The intensity of 13 Lysophosphatidylcholine (LPCs) and 2 glycerophosphocholines were lower in CHD patients (as shown in Fig.5).

To investigate latent relationships of those 109 significantly changed metabolites, spearman correlation analysis was also performed. Significantly changed plasma metabolites with smaller *adjustedp.value* either in CHD enriched metabolites or in control enriched metabolites had a relatively stronger correlation. Analysis among those 18 identified metabolites showed that LysoPC(18:0) had strong positive correlations with the following metabolites: LysoPC(18:0) vs LysoPC(P-16:0) *(rho = 0.861, q.value = 0),* LysoPC(20:3(5Z,8Z,11Z)) *(rho = 0.831, q.value* = 0), LysoPC(0:0/18:0) *(rho = 0.802, q.value* = *0)*. LysoPC(16:1(9Z)) had strong positive correlations with LysoPC(14:0) *(rho = 0.854, q.value* = 0) and LysoPC(18:0) *(rho= 0.815, q.value = 0).* On the other hand, L-Arginine negatively correlated with 1-Palmitoylglycerophosphocholine *(rho = -0.558, q.value = 1.07E-08).*

### Results for Urine Samples

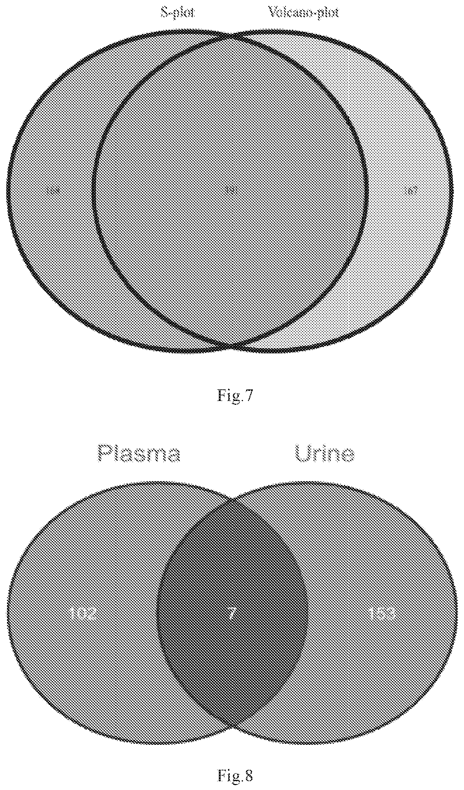

In the urine cloud plot (Fig.6), there were 870 m/z (30.44%) with increased intensity in CHD patients *(fold change > 1.2)* while the level of 557 m/z (19.49%) were decreased *(fold change < 0.8).* PCA and PLS-DA models were used and the analysis results were shown in PCA scores plot and three-dimensional PLS-DA scores plot *(PC1(4.34%), PC2(8.25%), PC3(2.99%)* ). This result indicated the CHD patients' urine samples were significantly different from healthy subjects'. PERMANOVA analysis demonstrated CHD had a significant impact on metabolic profile. Furthermore, S-plot analysis and Volcano-plot analysis were applied for potential biomarkers discovery. Using these criteria (*VIP >* 1, *adjusted p. value* produced by Mann-Whitney-Wilcoxon test after FDR correction < 0.05, *fold change* > 1.2 or < 0.8), 391 m/z were found to be significantly changed in CHD group by intersection of 558 m/z and 559 m/z in S-plot and Volcano-plot, respectively, as is shown in Veen plot (Fig.7).

The 391 m/z were aligned and annotated using the HMDB and KEGG database. Among the 160 annotated metabolites, the intensity of 96 metabolites were increased while that of 64 metabolites were decreased in CHD patients. These 160 metabolites were used to perform phenotype analysis for the 102 samples. The CHD patients' metabolism was obviously different from healthy controls. By comparing MS/MS spectra and retention time with commercially available reference standards, 4 metabolites were verified and the results were listed in Table 2. The level of GlcNAc-6-P and mannitol were increased with fold change of 165.99 and 8.45 in CHD patients respectively. Meanwhile, the level of creatine and phytosphingosine were decreased with fold changes of 0.41 and 0.39 respectively.

The level of GlcNAc-6-P was found to be increased in both plasma and urine samples. GlcNAc-6-P could be produced by human body enzymes and gut bacterial enzymes, so its origin need to be further determined. Similar pattern of GlcNAc-6-P increasing in urine and blood suggested interactions of host and gut flora were important in the development of CHD. Mannitol is a polyol or sugar alcohol produced by several microorganisms. The fact that no related homo sapiens enzymes are found in the fructose and mannose metabolism so far indicates mannitol might belong to microbial metabolites family. Mannitol could be produced by many microorganisms, such as lactic acid bacteria (Carvalheiro, F., Moniz, P., Duarte, L.C., Esteves, M.P. & Gírio, F.M. Mannitol production by lactic acid bacteria grown in supplemented carob syrup. J. Ind Microbiol. Biotechnol. 38, 221-7 (2011)) and pseudomonas putida (Kets, E.P.Galinski, E.A., de Wit, M., de Bont, J.A. &Heipieper, H.J. Mannitol, a novel bacterial compatible solute in Pseudomonas putida S12.J. Bacteriol.178, 6665-6670 (1996)). Creatine was also found to be decreased in CHD patient's urine sample. It is a nitrogenous organic acid naturally produced by the human body from amino acids. In biosystem, creatine can elevate creatine phosphate levels and improve maintenance of ATP content during tissue oxygen depletion period, and it also has the capacity to scavenge free radicals and reduce oxidative stress. Decreased creatine in CHD patients' urine indicated that obvious energy disturbance, more free radicals and more serious oxidative stress existed in CHD patients. Reduced phytosphingosine was also found in CHD patients' urine. Phytosphingosine is a phospholipid and a major component of mammalian tissue biological membranes. The synthesis of phytosphingosine can be performed by human body and intestinal microbiota in the sphingosine metabolism. Phytosphingosine could induce caspase-independent apoptosis in human T-cell lymphoma and non-small cell lung cancer cells. The decrease in urine phytosphingosine suggested sphingolipid metabolism was abnormal in CHD patients.

To evaluate correlation among 160 annotated urine metabolites, spearman correlation analysis was performed. The results showed that urine metabolites which were significantly changed (with smaller *adjustedp.value)* had relatively stronger correlations compared with plasma significant metabolites. In addition, among those 4 validated metabolites, mannitol showed a relatively high positive correlation with GlcNAc-6-P *(rho = 0.775, q.value =9.40E-*21) and this could be a signal for disturbed intestine microbiota.

### Correlations between plasma and urine significant metabolites.

To apply metabolomics to clinical diagnosis, treatments or pathophysiology research, physiological function of metabolites and relationships between them needed to be illustrated, and correlation networks of all potential biomarkers needed to be built (Liu, P. et al. Biomarkers of primary dysmenorrhea and herbal formula intervention: an exploratory metabonomics study of blood plasma and urine. Mol. BioSyst.9, 77-87 (2013)). For this purpose, the significantly changed plasma and urine metabolites were connected according to spearman correlation analysis profiled with Cytoscape software. These 109 annotated significantly changed plasma metabolites and 160 annotated significantly changed urine metabolites were involved in different pathways and can be divided into 8 categories: carbohydrate metabolism, lipids metabolism, amino acids metabolism, bile acids metabolism, purine/pyrimidine metabolism, vitamins metabolism, microbial related metabolism and others. Lipids metabolism showed significantly negatively correlations with microbial related metabolism while other 6 metabolism categories were in strong positive correlation with microbial related metabolism.

Seven significantly changed metabolites (Supplementary Table 2), including GlcNAc-6-P, were found both in plasma and urine on the condition that retention time error was less than 1 min and m/z error was less than 0.01 Dalton with MS/MS comparison. A Veen diagram exhibiting the common metabolites among plasma and urine significantly changed metabolites is provided in Fig.8. Two metabolites (m/z: 185.04, 202.04) were decreased in CHD patients while other five metabolites (m/z: 125.01, 309.05, 310.04, 311.05, 324.04) were increased in CHD patients.

To evaluate the correlation among 7 common metabolites, spearman correlation analysis was implemented using the criteria that the coefficient was larger than 0.90. First, correlations among plasma metabolites were obtained, m/z 311.05 showed strong correlation with m/z 309.05 *(rho = 0.929, q. value = 9.31E-45),* m/z 310.04 *(rho = 0.911, q. value = 5.70E-40),* m/z 324.04 *(rho = 0.900, q.value = 1.53E-37);* m/z 309.05 also strongly correlated with m/z 310.04 *(rho = 0.929, q.value = 9.31E-45).* Second, correlations among urine metabolites were obtained, GlcNAc-6-P (m/z 324.04) was strongly correlated with m/z 310.04 *(rho = 0.933, q.value = 1.26E-45),* m/z 311.05 *(rho = 0.910, q.value = 1.17E-39),* m/z 125.01 *(rho = 0.903, q.value = 3.43E-38),* while m/z 125.01 also showed strong correlation with urine metabolite (m/z 310.04 *(rho = 0.918, q.value = 1.28E-41).* In addition, correlations of these metabolites in plasma and urine were also evaluated. The results showed that plasma metabolites have strong positive correlations with the same metabolites in urine (Supplementary Table3). Among them, validated GlcNAc-6-P (324.04) showed very strong positive correlation with itself *(rho = 0.747, q.value = 5.60E-19).*

### Clinical relevance of plasma and urine potential metabolites

### Receiver operating characteristic analysis

To evaluate the potential of the identified metabolites (18 plasma and 4 urine ones) as biomarkers, receiver operating characteristic analysis (ROC) was applied to 176 additional plasma samples (98 healthy controls vs 78 CHD patients) and 395 additional urine samples (173 healthy controls vs 222 CHD patients).

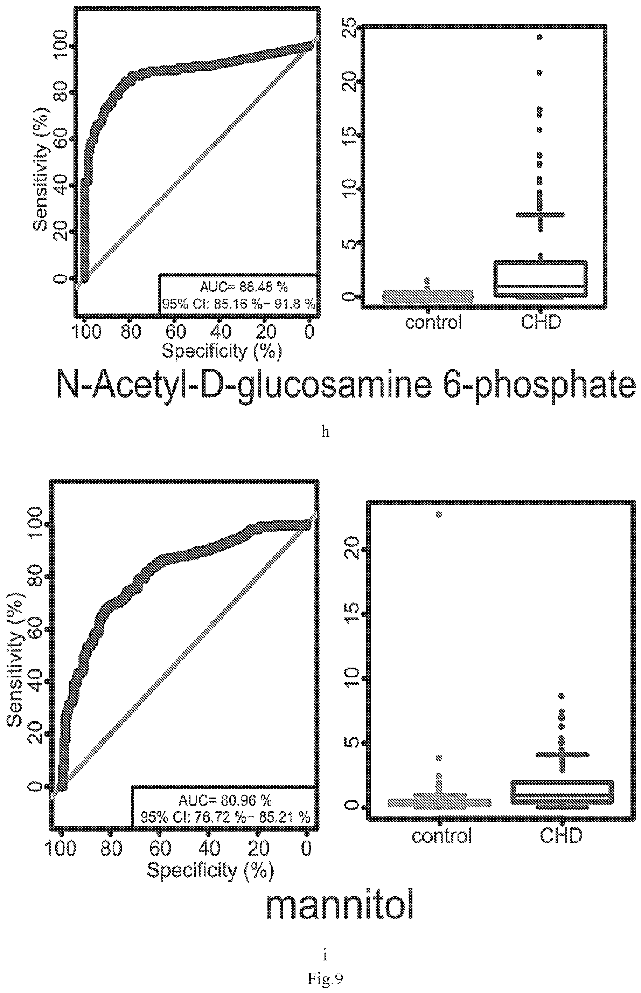

In plasma validation datasets, 6 LPCs and 1 glycerophosphocholine metabolites showed area under curve *(AUC)* larger than 0.80 and were significantly different in CHD patients (Table 3). As shown in Fig. 9a-g, The levels of LysoPC(18:3(6Z,9Z,12Z)), LysoPC(P-16:0), LysoPC(15:0), 1-Palmitoylglycerophosphocholine, LysoPC(14:0), LysoPC(16:1(9Z)), LysoPC(0:0/18:0) were decreased in CHD patients with *fold change* at 0.26, 0.58, 0.51, 0.65, 0.49, 0.62, 0.42 respectively *and 4 PC* of 0.91, 0.88, 0.88, 0.88, 0.84, 0.83, 0.83 respectively. On the other hand, other 9 plasma potential biomarkers exhibited the same enrichment direction except that LysoPC(20:3(5Z,8Z,11Z)) became normal and GlcNAc-6-P even became undetected (data shown in Table 3). These results validated that LPCs could become biomarkers and targets for CHD diagnosis and therapies in the future.

In urine validation datasets, GlcNAc-6-P and mannitol exhibited *A UC* of 0.88, 0.81 and *fold change* at 36.91 and 2.62 respectively (as shown in Fig. 9h, 9i and Table 4). However, creatine and phytosphingosine did not show good diagnostic ability in both training and validation datasets. The existence of GlcNAc-6-P and mannitol in urine indicates the interaction of gut flora activity and host metabolism. These results also validated that GlcNAc-6-P and mannitol could become biomarkers and targets for CHD diagnosis and therapies in the future.

Among these 7 choline metabolites and 2 urine metabolites with *AUC* larger than 0.80, GlcNAc-6-P appeared the most discriminative biomarker which showed relatively good diagnostic ability with false negative (*FN*) of 0.051, 0.153 and false positive (FP) of 0.047, 0.208 in the training datasets and validation datasets respectively. LysoPC(18:3(6Z,9Z,12Z)), LysoPC(P-16:0), LysoPC(15:0), 1-Palmitoylglycerophosphocholine, LysoPC(14:0), LysoPC(16:1(9Z)), LysoPC(0:0/18:0) and mannitol exhibited diagnostic ability with *FN* of 0.271, 0.169, 0.136, 0.068, 0.119, 0.119, 0.085, 0.153 and *FP* of 0.233, 0.163, 0.256, 0.233, 0.209, 0.140, 0.279, 0.093 respectively in the training datasets, showed diagnostic ability with *FN* of 0.013, 0, 0, 0.013, 0.013, 0.013, 0, 0.135 and *FP* of 0.582, 0.755, 0.673, 0.694, 0.612, 0.684, 0.714, 0.416 respectively in the validation datasets. The *FN* and *FP* of 7 choline metabolites and 2 urine metabolites were all analysed with R using the "randomForest" and "pROC" packages based on the intensity of training datasets shown in Table 7 and Table 8 respectively. The randomForest model classification output prediction results (probability of illness; cutoff was 0.5, and if the probability of illness ≥0.5, the subject was at risk of CHD).

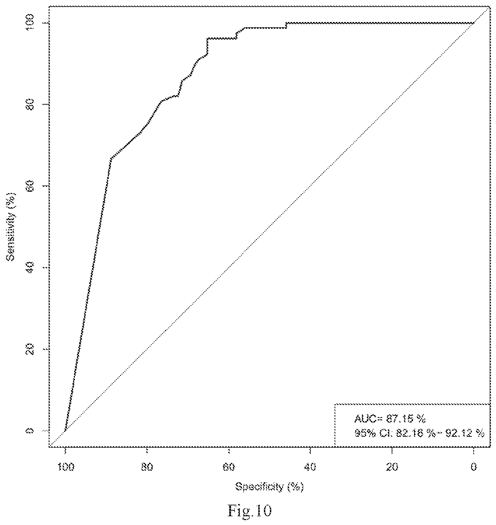

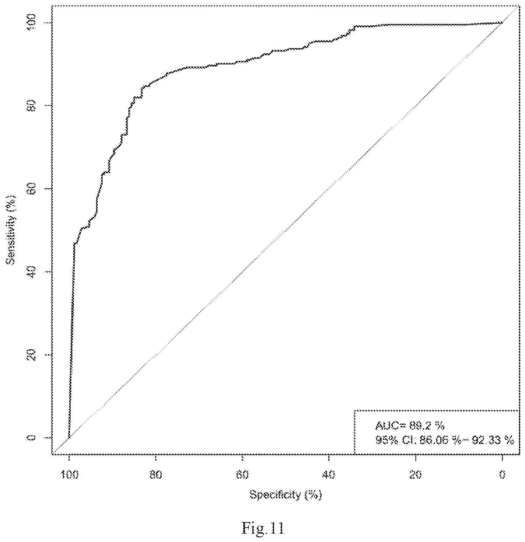

7 potential plasma biomarkers were combined to perform ROC analysis in plasma training datasets (59 CHD patients plasma samples VS 43 healthy control plasma samples) and plasma validation datasets (78 CHD patients plasma samples VS 98 healthy controls plasma samples, Fig. 10, Table 9, Table 11) with R package - pROC. The FN and FP were (0,0) and (0,0.724) in plasma training and validation datasets respectively. 2 potential urine biomarkers were combined to perform ROC analysis in urine training datasets (59 CHD patients urine samples VS 43 healthy control urine samples) and urine validation datasets (222 CHD patients urine samples VS 173 healthy controls urine samples, Fig. 11, Table 10, Table 11) with R package - pROC. The FN and FP were (0.016,0) and (0.121,0.225) in urine training and validation datasets respectively. The FN and FP of 7 choline metabolites combination and 2 urine metabolites combination were all analysed with R using the "randomForest" and "pROC" packages based on the intensity of training datasets shown in Table 7 and Table 8 respectively. The randomForest model classification output prediction results (probability of illness; cutoff was 0.5, and if the probability of illness ≥0.5, the subject was at risk of CHD).

**Table 8 Ion intensity of 2 urine biomarkers in training datasets**

| 102 samples(CD:CHD patients;N: healthy controls) | Mannitol | N-Acetyl-D-glucosamine 6-phosphate(urine) |
|---|---|---|
| CD6582 | 1.797061721 | 2.008409683 |
| CD6584 | 0.226624013 | 0.10327254 |
| CD6586 | 2.710241568 | 0.884830292 |
| CD6591 | 1.896246116 | 4.657566027 |
| CD6594 | 2.409460936 | 0.165203306 |
| CD6595 | 4.227845648 | 0.89311435 |
| CD6598 | 2.300193588 | 2.584746942 |
| CD6601 | 5.041102453 | 1.5649008 |
| CD6604 | 0.174956189 | 0.132183533 |
| CD6608 | 2.415311223 | 0.695486472 |
| CD6610 | 2.672194945 | 4.230489787 |
| CD6620 | 6.387234607 | 1.802220937 |
| CD6636 | 3.006253932 | 1.314180932 |
| CD6643 | 3.292375427 | 0.961902492 |
| CD6644 | 1.200196587 | 3.115646653 |
| CD6645 | 1.462288938 | 0.797846386 |
| CD6647 | 0.251544654 | 0.279477761 |
| CD6649 | 2.713858641 | 1.615554991 |
| CD6650 | 0.652042092 | 0.044490965 |
| CD6651 | 0.752386394 | 1.859552244 |
| CD6654 | 4.566307634 | 0.504933042 |
| CD6657 | 0.568049146 | 0.164254784 |
| CD6700 | 0.143825449 | 0.458631745 |
| CD6703 | 2.401161313 | 2.159423374 |
| CD6716 | 2.371362643 | 5.123717293 |
| CD6722 | 0.513280653 | 0.244211889 |
| CD6733 | 0.524881048 | 0.072688105 |
| CD8049 | 1.50557905 | 1.268546604 |
| CD8051 | 1.558284587 | 2.145153427 |
| CD8059 | 0.769880388 | 2.625018896 |
| CD8117 | 0.228313759 | 0 |
| CD8118 | 3.382564216 | 4.106515516 |
| CD8119 | 0.068429132 | 0.000485643 |
| CD8122 | 1.221847669 | 0.048104832 |
| CD8144 | 0.410650908 | 0.955945844 |
| CD8145 | 0.444317971 | 0.478727164 |
| CD8149 | 0.286118413 | 0.432262471 |
| CD8185 | 2.508419073 | 3.831520076 |
| CD8196 | 2.758853927 | 1.324228171 |
| CD8197 | 1.349624108 | 1.58524808 |
| CD8203 | 2.723637075 | 3.86801429 |
| CD8211 | 3.007940982 | 1.076172348 |
| CD8212 | 1.551084517 | 0.837872241 |
| CD8234 | 0.529333184 | 0.471319856 |
| CD8239 | 1.387151665 | 1.670098916 |
| CD8243 | 4.341736372 | 1.172368242 |
| CD8257 | 0.116144417 | 0.604577262 |
| CD8264 | 0.34357398 | 0.064247251 |
| CD8305 | 2.680379198 | 1.57681171 |
| CD8313 | 0.968655789 | 0.613067188 |
| CD8321 | 0.386851883 | 0 |
| CD8330 | 1.93447556 | 0.863686633 |
| CD8331 | 0.331465917 | 0.627168749 |
| CD8350 | 2.623385724 | 2.895039926 |
| CD8355 | 3.073236226 | 7.017030406 |
| CD8367 | 1.477988579 | 2.034458376 |
| CD8368 | 3.411880495 | 0.242533069 |
| CD8376 | 2.622249212 | 0.915733388 |
| CD8380 | 2.682825895 | 2.690576715 |
| N1260 | 0.069542295 | 0 |
| N1261 | 0.114644304 | 0.000801217 |
| N1262 | 0.08888305 | 0 |
| N1263 | 0.068171425 | 0.002070223 |
| N1264 | 0.189242942 | 0 |
| N1265 | 0.180078358 | 0.000706783 |
| N1266 | 0.121853185 | 0.27344985 |
| N1267 | 0.180933418 | 0 |
| N1268 | 0.165977157 | 0 |
| N1269 | 0.103173296 | 0 |
| N1270 | 0.635201398 | 0 |
| N1271 | 0.192760107 | 0.007115928 |
| N1272 | 0.140730645 | 0 |
| N1273 | 0.146370371 | 0 |
| N1274 | 0.137038642 | 0 |
| N1275 | 0.215131448 | 0.000326432 |
| N1276 | 0.592933662 | 0 |
| N1277 | 0.152641519 | 0.002643811 |
| N1279 | 0.094234335 | 0.000564699 |
| N1280 | 0.099827238 | 0 |
| N1282 | 0.268085416 | 0 |
| N1283 | 0.146320383 | 0 |
| N1284 | 0.259701183 | 0.003914832 |
| N1285 | 0.091652296 | 0.000684173 |
| N1286 | 0.237952959 | 0.00263401 |
| N1287 | 0.174966198 | 0 |
| N1288 | 0.338345326 | 0.019954638 |
| N1290 | 0.144976064 | 0.000768401 |
| N1291 | 0.199915016 | 0.000789679 |
| N1292 | 0.132424354 | 0.001558165 |
| N1293 | 0.530116055 | 0.000412933 |
| N1294 | 0.292173012 | 0.000716079 |
| N1295 | 0.186541303 | 0.00124535 |
| N1296 | 0.15838471 | 0 |
| N1297 | 0.123834589 | 0 |
| N1298 | 0.221444982 | 0 |
| N1299 | 0.285606734 | 0.05470694 |
| N1300 | 0.340578027 | 0.001896021 |
| N1301 | 0.231590095 | 0 |
| N1302 | 0.110948604 | 0 |
| N1307 | 0.864877643 | 0.000980615 |
| N1308 | 0.076948403 | 0 |
| N1309 | 0.318979223 | 0.001756577 |

**Table 9 Ion intensity of 7 plasma biomarkers in two samples of validation datasets**

| samples | label (1:CHD patients, 0:healt hy individuals) | LysoPC (14:0) | LysoPC (15:0) | LysoPC(16 :1(9Z)) | LysoPC(18:3(6 Z,9Z,12Z)) | 1-Palmitoylglyceropho sphocholine | LysoPC( P-16:0) | LysoPC(0: 0/18:0) |
|---|---|---|---|---|---|---|---|---|
| N190E_2 | 0 | 1.78968 0525 | 1.52850 4271 | 1.5644254 02 | 0.928544121 | 1.332653635 | 1.383823 769 | 1.2018002 54 |
| CD5751_1 | 1 | 1.21255 2792 | 1.07035 4256 | 0.8414078 44 | 0.455679314 | 0.882024927 | 0.790375 507 | 0.6154920 81 |

**Table 10 Ion intensity of 2 urine biomarkers in two samples of validation datasets**

| samples | label (1:CHD patients; 0: healthy individuals) | Mannitol | N-Acetyl-D-glucosamine-6-phosphate |
|---|---|---|---|
| N003_6_6 | 0 | 0.3165 | 0 |
| ZSL001_9_8 | 1 | 0.403944 | 0.309672 |

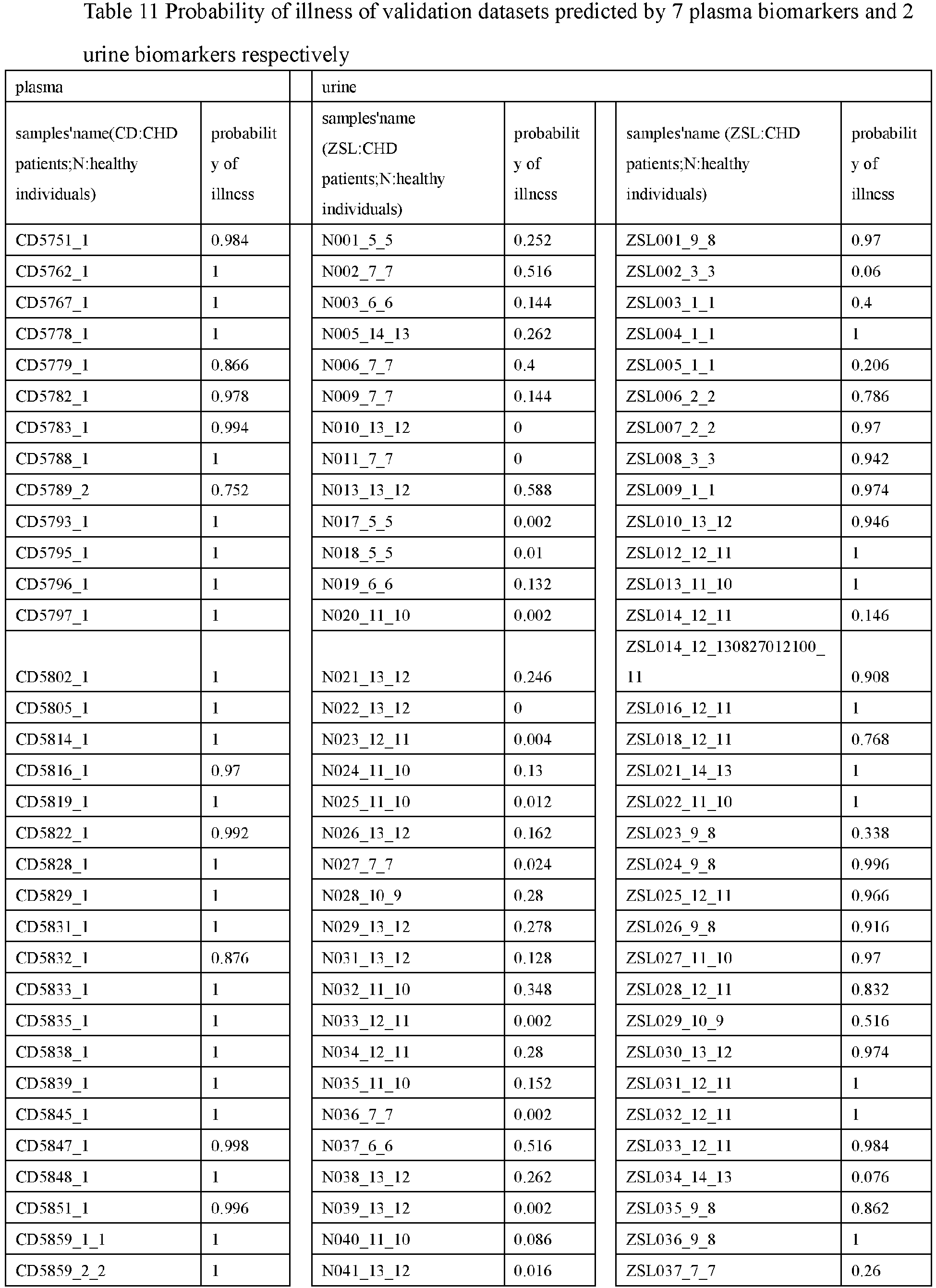

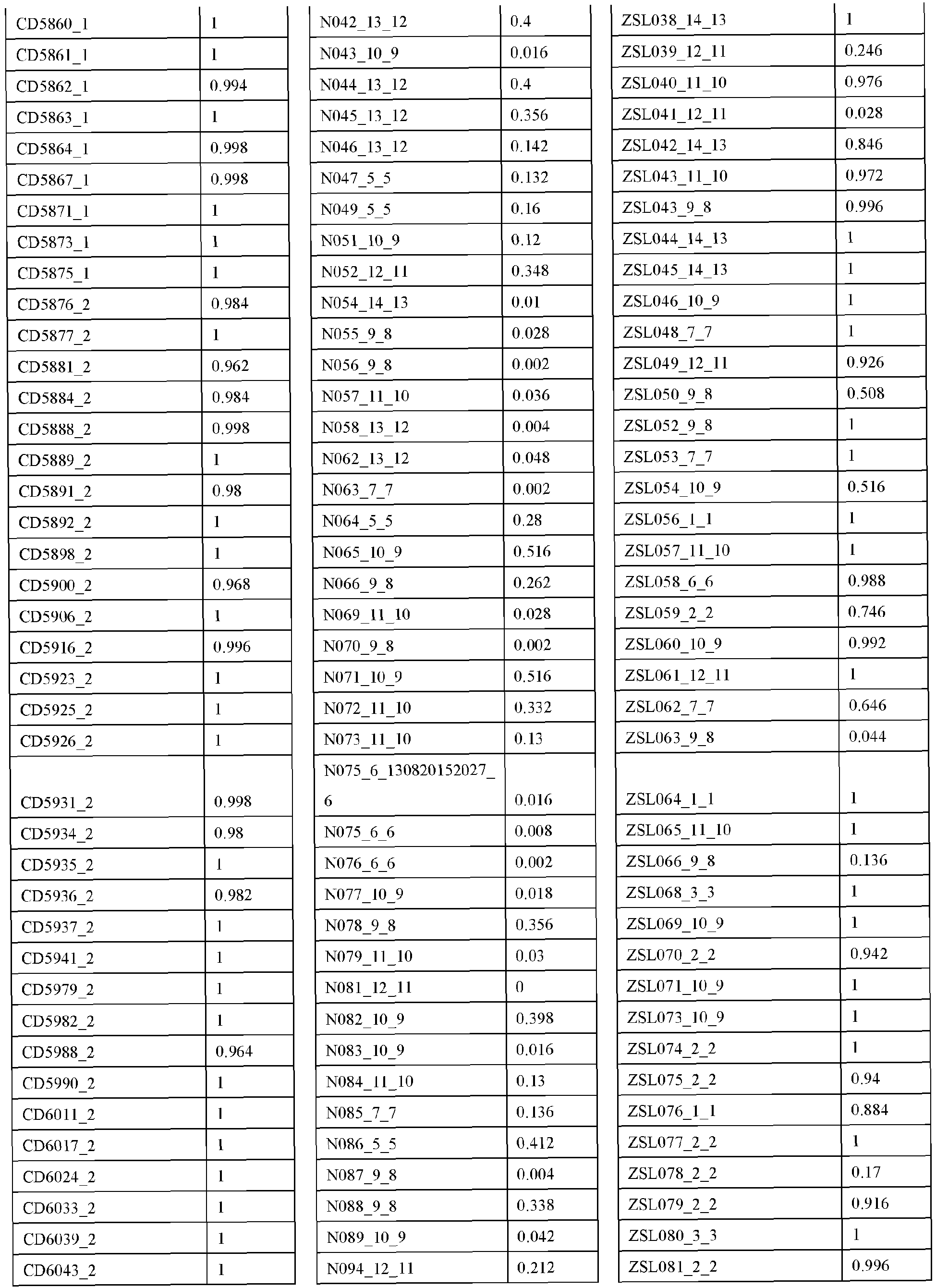

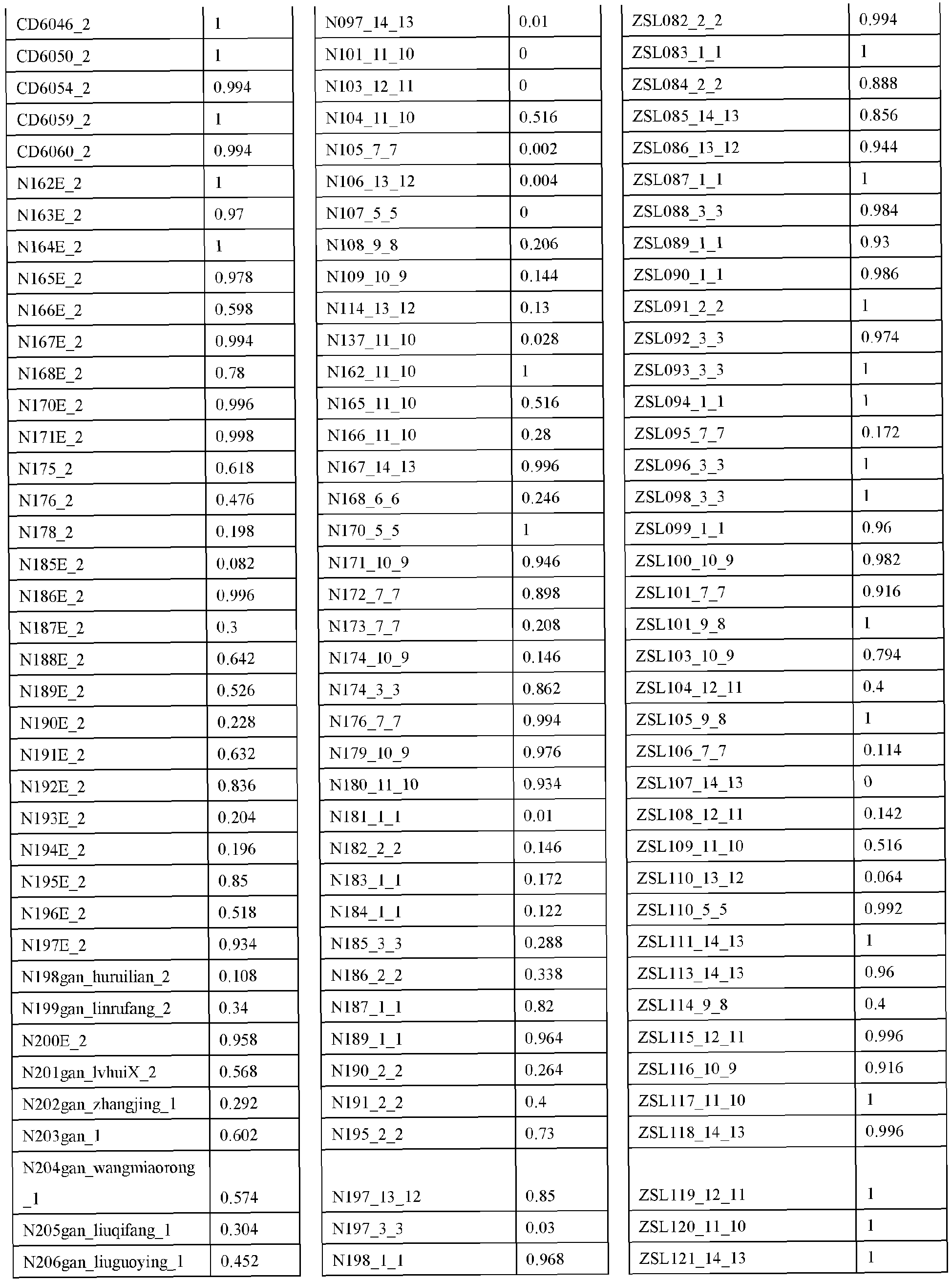

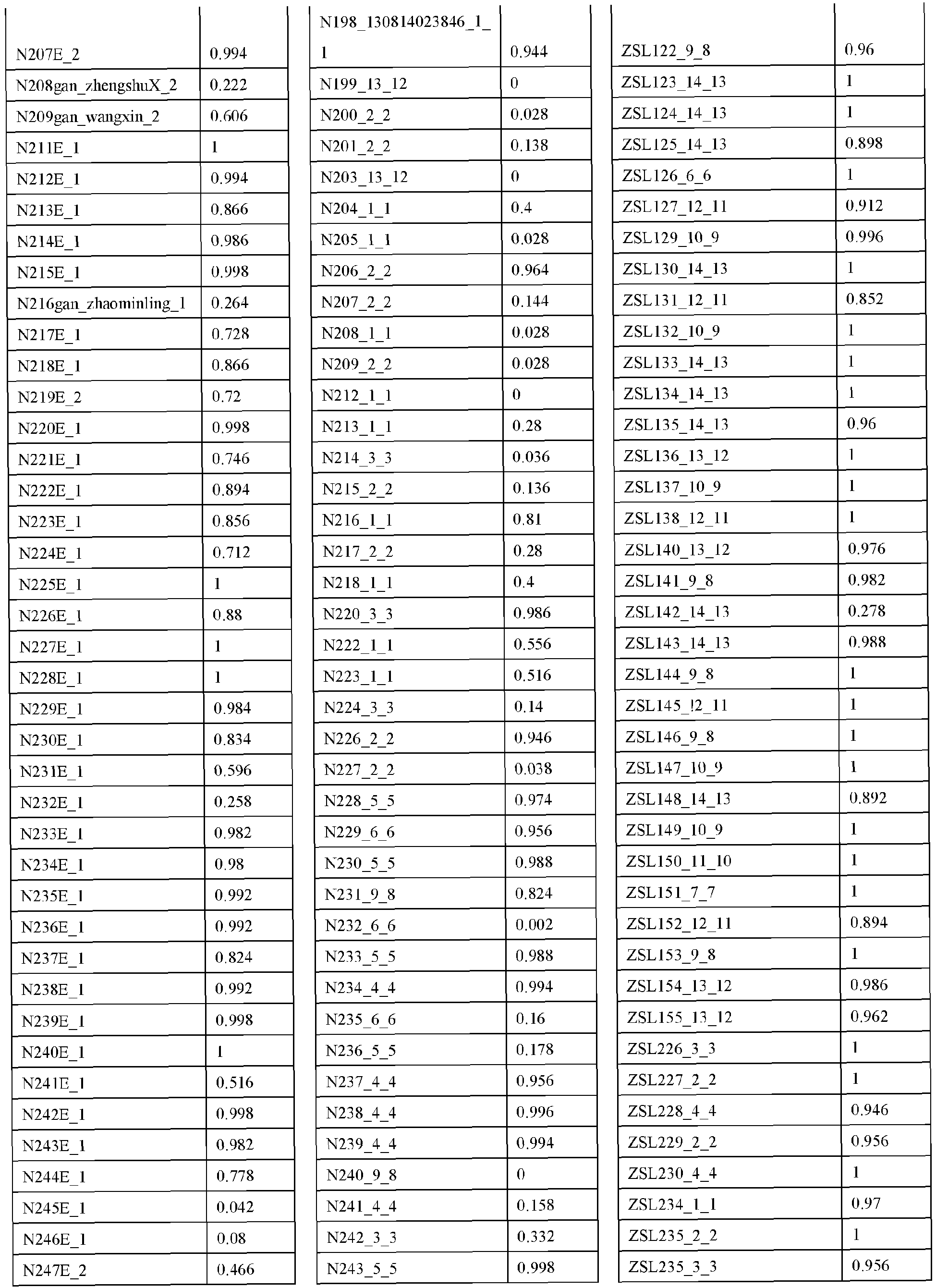

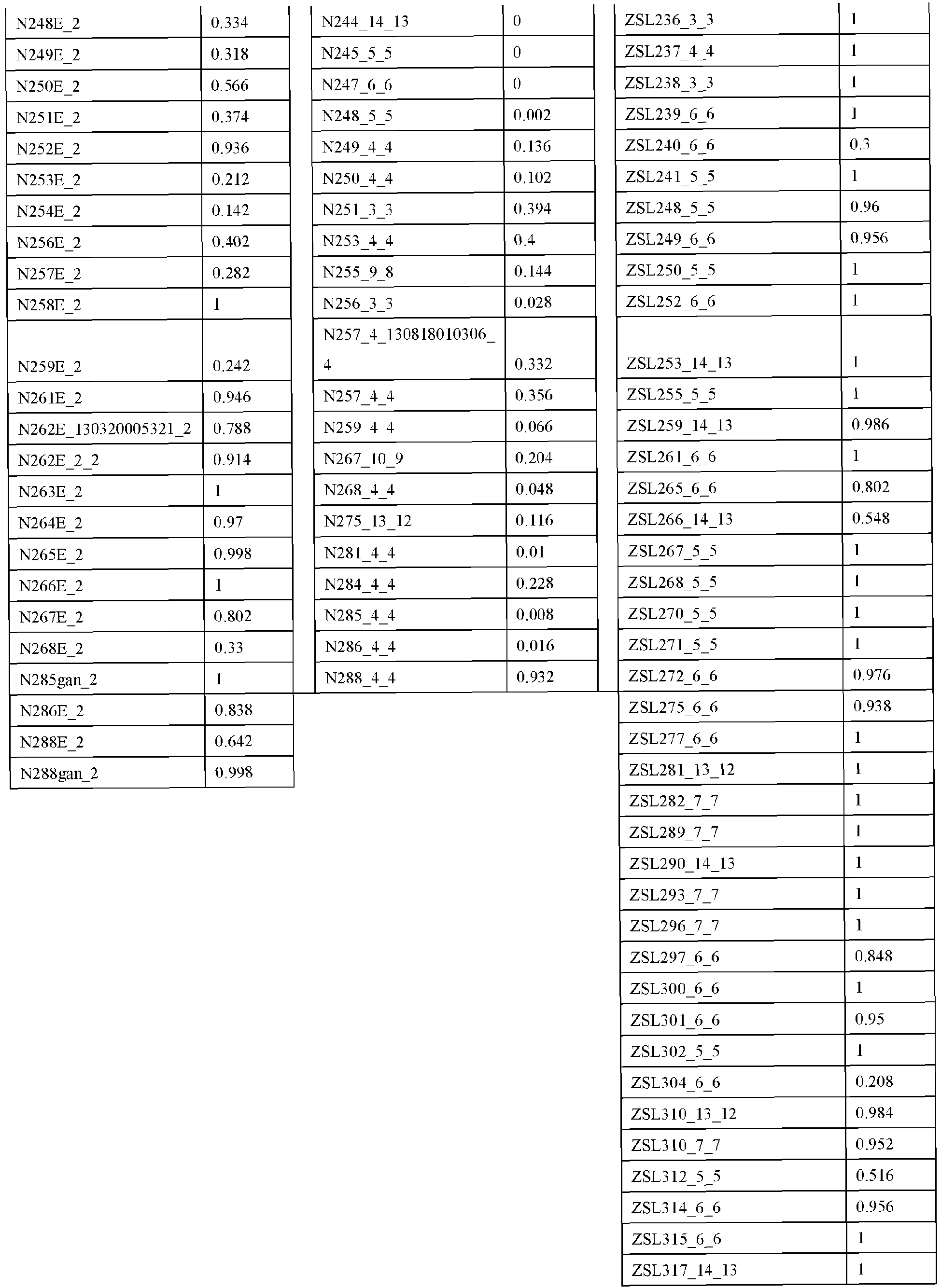

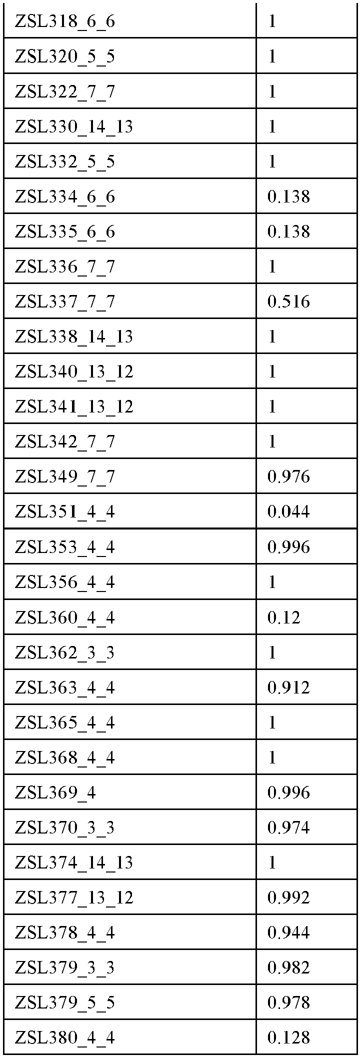

### Example 2. Association of potential metabolic biomarkers with clinical phenotypes

To access the effects of patients' covariates (such as age and clinical biochemical factors) on metabolic profiles, PERMANOVA analysis was performed. Albumin (ALB), alanine aminotransferase (ALT), total protein (TP), low-density lipoprotein (LDLC), cholesterol (CHOL), high-density lipoprotein (HDLC), apolipoprotein b (APOB) and apolipoprotein a (APOA) were found to be significantly different in CHD patients (Supplementary Table 1).

Besides, spearman correlation analysis was performed among 18 potential plasma biomarkers and 4 potential urine biomarkers with individual phenotypes. CHOL, HDLC and TP showed significantly positive correlations with plasma LPCs (Supplementary Table 4).

LysoPC (18:0) was correlated with CHOL *(rho = 0.518, q.value = 7.89E-07),* HDLC *(rho = 0.548, q.value = 1.29E-07)* and TP *(rho = 0.573, q.value = 5.16E-08).* LysoPC(P-16:0) was positively correlated with HDLC *(rho = 0.561, q.value* = 7.39E-08). Meanwhile, the two potential urine biomarkers, GlcNAc-6-P and mannitol, exhibited strong negative correlations with CHOL, HDLC, TP and APOB *(q.value <* 0.01). These results indicated significantly abnormal LPCs metabolism in CHD patients, and thus we speculated that it could be beneficial to reduce CHD occurrence by properly increasing intake of these extra LPCs which were significantly decreased in CHD patients.

### Example 3. Gut flora associated potential biomarkers

**DNA extraction from fecal samples.** 102 Fecal samples (mentioned in Example 1) were thawed on ice and DNA was extracted using the Qiagen QIAamp DNA Stool Mini Kit (Qiagen) according to manufacturer's instructions. Extracts were treated with DNase-free RNase to eliminate RNA contamination. And DNA quantity was measured using NanoDrop spectrophotometer, Qubit Fluorometer (with the Quant-iTTMdsDNA BR Assay Kit) and gel electrophoresis.

**DNA library construction and metagenomic sequencing of fecal samples.** DNA library construction was performed following the manufacturer's instruction (Illumina). The detailed method was described previously (Qin, J. et al. A metagenome-wide association study of gut microbiota in type 2 diabetes. Nature 490, 55-60, doi:10.1038/nature11450 (2012)). Basically, the following procedures were performed: cluster generation, template hybridization, isothermal amplification, linearization, blocking and denaturation, and hybridization of the sequencing primers. One paired-end (PE) library was constructed with insert size of 350 bp for each sample, followed by a high-throughput sequencing to obtain around 30 million PE reads with a length of 2x100bp. High-quality reads were obtained by filtering low-quality reads with ambiguous 'N' bases, adapter contamination and human DNA contamination from the Illumina raw reads, and by trimming low-quality terminal bases of reads simultaneously.

**Gene catalogue construction.** For the sequencing reads of another new 314 samples(159 CHD patients vs 155 controls), the employed parameters were the same as previous publication (Qin et al. 2012, *supra),* de novo assembly and gene prediction was performed using SOAPdenovo v1.06 (Li, R. et al. De novo assembly of human genomes with massively parallel short read sequencing. Genome Research 20, 265-272, doi:10.1101/gr.097261.109 (2009)) and GeneMark v2.7 (Zhu, W., Lomsadze, A. & Borodovsky, M. Ab initio gene identification in metagenomic sequences. Nucleic acids research 38, e132, doi:10.1093/nar/gkq275 (2010).35. Sunagawa, S. et al. Metagenomic species profiling using universal phylogenetic marker genes. Nature methods 10, 1196-1199 (2013)) softwares, respectively. All predicted genes were aligned pairwise using BLAT. Redundant genes were removed using BLAT with the cutoff of 90% overlap and 95% identity (no gaps allowed), resulting in a non-redundant gene catalogue comprising of 4,537,046 genes (4.5 M gene catalogue).

**Taxonomic assignment of genes.** Taxonomic assignment of the predicted genes was performed using an in-house pipeline which was described in previous publication (Qin et al. 2012, *supra),* with 80% overlap and 65% identity top 10% scores (BLASTN v2.2.24, -e 0.01 - b 100 -K 1 -F T -m 8). The cutoffs were 65% identity for assignment to phylum, 85% identity to genus, 95% identity to species and ≥ 50% consensus for the taxon under question, if multiple hits remained.

**Functional annotation.** Putative amino acid sequences, which translated from our gene catalogue, were aligned against the proteins/domains in KEGG databases (release 59.0) using BLASTP (e-value ≤ 1e-5). Each protein was assigned to the KEGG orthologue group (KO) by the highest scoring annotated hit(s) containing at least one HSP scoring over 60 bits.

**Species profiles.** Total fecal clean reads were aligned to the 79268 sequences of mOTU reference (Sunagawa, S. et al. Metagenomic species profiling using universal phylogenetic marker genes. Nature methods 10, 1196-1199 (2013)) with default parameters. 512 species level were identified. The inventors developed a method that uses a subset of marker genes (MGs) for taxonomic profiling of metagenomes (Sunagawa et al., 2013). It is available as a standalone software and is also implemented in MOCAT. Species-level profiles (relative abundances) are generated by mapping reads from metagenomes to a database (mOTU.v1.padded) consisting of 10 MGs extracted from 3,496 prokaryotic reference genomes (downloaded from NCBI) and 263 publicly available metagenomes (from the MetaHIT and HMP projects).

### Gut flora associated potential biomarkers

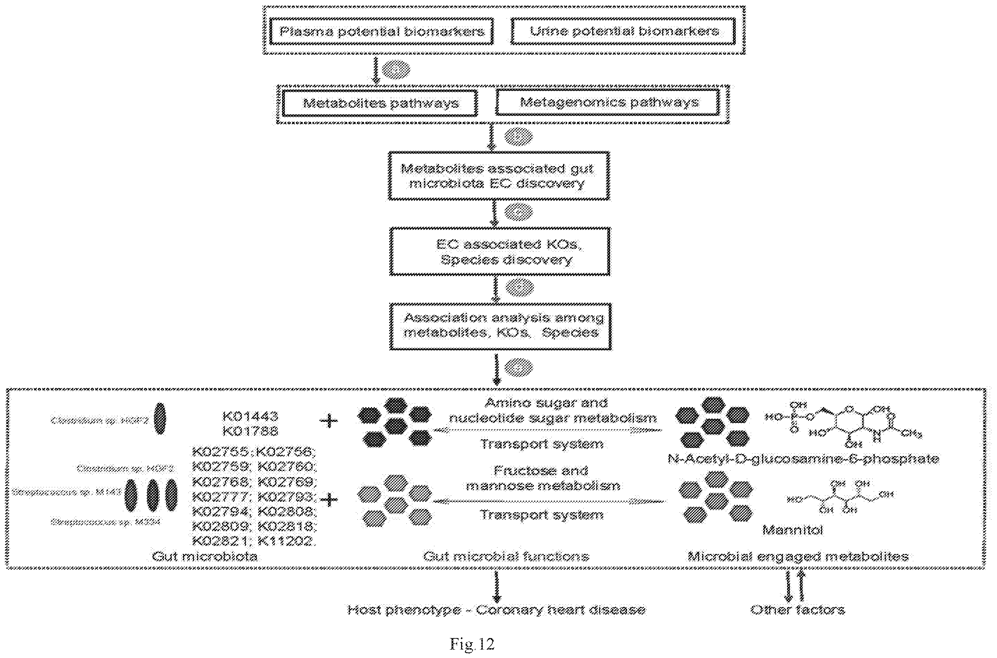

Human body is a complex biosystem with numerous co-existing microbial species. Previous study suggests that around 30% of metabolites detected in human body originate from microbiota (E Holmes, JV Li, JR Marchesi, et al. Gut Microbiota Composition and Activity in Relation to Host Metabolic Phenotype and Disease Risk[J]. Cell Metabolism. 2012 Nov; 15(5): 559-564). In this study, pathway analysis for plasma and urine metabolites indicates that some potential biomarkers like GlcNAc-6-P and Mannitol could be of microbial origin. To discover gut flora species significantly associated with the identified potential biomarkers, integrated analysis of metabolomics and metagenomics were performed for the patient and control groups, as shown in Fig.12. Metabolite associated gut microbiota ECs (Enzyme Commission) were analyzed and some significantly changed ECs were found to be engaged in metabolic pathways, such as amino sugar and nucleotide sugar metabolism, arginine and proline metabolism, glycerophospholipid metabolism, fructose and mannose metabolism. Based on integrated analysis of metabolites pathways and gut microbiota participated pathways, these gut microbial ECs seem to affect the productions and functions of those identified potential biomarkers.

Analysis of EC associated with significantly changed metabolites in plasma and urine showed the following results: the significantly changed ECs associated with GlcNAc-6-P were EC(2.7.1.69), EC(2.7.1.59), EC(3.2.1.14), EC(5.1.3.9), EC(2.7.1.60), EC(3.5.1.25), EC(5.4.2.10), EC(2.3.1.157), EC(2.7.7.23) and EC(4.1.3.3). Meanwhile, EC(3.5.2.10) was associated with creatine ; EC(1.1.1.14), EC(2.7.1.69) and EC(3.2.1.80) were associated with mannitol; EC(3.5.3.6), EC(2.1.3.3), and EC(2.1.3.9) were associated with arginine; EC(3.1.1.5) and EC(3.1.1.32) were correlated with LPCs.

These significantly changed ECs were then annotated with 65 KOs. And spearman correlation analysis was applied to these 65 KOs and the total 22 identified potential plasma and urine metabolites biomarkers (18 plasma ones and 4 urine ones listed in Table 1 and 2). The results showed that 16 CHD enriched KOs were significantly correlated with GlcNAc-6-P (both in plasma and urine) and mannitol (in urine) (Supplementary Table 6).

Besides, to identify the microbial species correlated with the 22 significantly changed metabolites, spearman correlation analysis of 512 annotated species with those 22 biomarkers was also implemented (Table 5). One gut flora species - *Clostridium sp. HGF2 (p.value =* 9.86E-05, *q. value =* 8.65E-03, NCBI Reference Sequence: NZ_AENW00000000.1), was found to positively significantly correlate with GlcNAc-6-P while three gut flora species - *Streptococcus sp. M334 (p.value* = 3.13E-02, *q.value* = 2.39E-01), *Streptococcus sp. M143 (p.value* = 3.49E-02, *q.value* = 2.40E-01), and *Clostridium sp. HGF2 (p.value* = 9.86E-05, *q.value* = 8.65E-03) were positively correlated with mannitol. The above three gut flora species were CHD enriched changed gut microbiota. Interestingly, *Clostridium sp. HGF2* positively associated with both GlcNAc-6-P and mannitol. By combining the association results of KOs and flora species with those 22 identified potential biomarkers, we found that *Streptococcus sp. M334* and *M143, Clostridium sp. HGF2* and their associated metabolites were involved in the development of coronary heart disease. This study provides the first direct evidence that microbial metabolites are involved in the CHD disease and identified the specific flora species regulating the microbial metabolites detected in plasma and urine of CHD patients. These results can lead to a new strategy for CHD treatments: adjusting the activity of these changed microbial species and the overall flora ecological environment in patients.

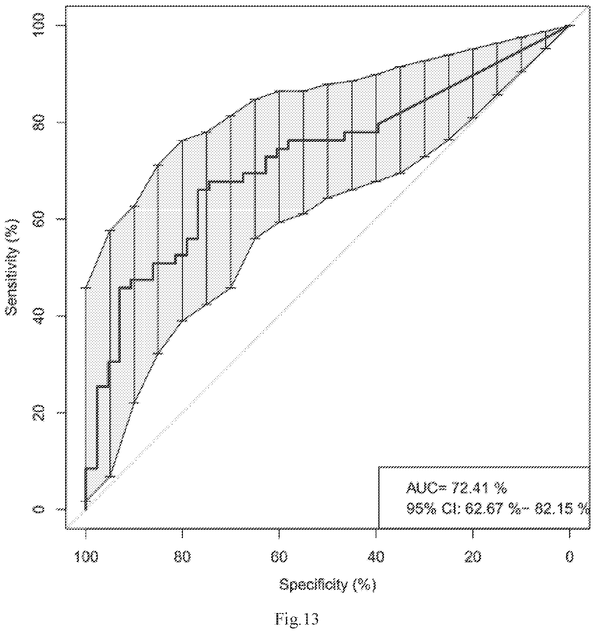

Using relative abundances of *Clostridium sp.* HGF2 as the risk score (Table 13), the inventors performed ROC analysis in another new fecal samples. For *Clostridium sp.* HGF2, the inventors can estimate an AUC and its best cutoff where the sum of the prediction sensitivity and specificity reaches its maximum (Fig.13, Table 12). If the sample's relative abundances of *Clostridium sp.* HGF2 was larger than the best cutoff, then the person was at CHD risk.

**Table 12 AUC and best cutoff of Clostridium sp. HGF2**

| best cutoff (relative abundances) | FPR (false positive rate) | TPR (true positive rate) | AUC |
|---|---|---|---|
| 0.00012083 | 0.2325581395 | 0.6610169492 | 0.7240835633 |

**Table 13 Relative abundances of Clostridium sp. HGF2 in fecal samples**

| Samples (VZSL:CHD patients;VN:healthy individuals) | Clostridium sp. HGF2 |
|---|---|
| VZSL399 | 0.000367357 |
| VZSL401 | 0.000115793 |
| VZSL402 | 0.000150153 |
| VZSL403 | 0.000978874 |
| VZSL404 | 0.000767806 |
| VZSL405 | 0.000974222 |
| VZSL408 | 0.000563463 |
| VZSL409 | 0.007103486 |
| VZSL415 | 0.00188733 |
| VZSL416 | 0 |
| VZSL417 | 0.000261392 |
| VZSL422 | 0.000169998 |
| VZSL429 | 0 |
| VZSL430 | 9.22E-05 |
| VZSL431 | 0 |
| VZSL432 | 0 |
| VZSL433 | 9.74E-05 |
| VZSL435 | 0 |
| VZSL436 | 0.000247886 |
| VZSL437 | 0.008548325 |
| VZSL439 | 0.000340268 |
| VZSL444 | 0 |
| VZSL446 | 0.000257002 |
| VZSL450 | 0.008210507 |
| VZSL452 | 0.00022191 |
| VZSL453 | 0.000975159 |
| VZSL454 | 0.000135128 |
| VZSL466 | 0 |
| VZSL467 | 0.000278775 |
| VZSL468 | 0 |
| VZSL475 | 0.000476429 |
| VZSL477 | 4.94E-05 |
| VZSL478 | 0.000592479 |
| VZSL479 | 8.40E-05 |
| VZSL480 | 0.000517041 |
| VZSL482 | 0.000121481 |
| VZSL487 | 0 |
| VZSL488 | 0.00018343 |
| VZSL490 | 9.71E-05 |
| VZSL492 | 0.00096547 |
| VZSL495 | 0.000163234 |
| VZSL496 | 0.000122875 |
| VZSL497 | 0.000121312 |
| VZSL501 | 0.000933894 |
| VZSL503 | 0.000841729 |
| VZSL505 | 0.0001492 |
| VZSL506 | 0.000120838 |
| VZSL510 | 0.000323609 |
| VZSL512 | 0.012278164 |
| VZSL515 | 0 |
| VZSL516 | 0.002028835 |
| VZSL517 | 9.12E-05 |
| VZSL520 | 0 |
| VZSL524 | 0.000842572 |
| VZSL525 | 0.000347477 |
| VZSL527 | 0.000101661 |
| VZSL529 | 0.017505533 |
| VZSL530 | 0 |
| VZSL532 | 0.000137125 |
| VN1260 | 0.000200507 |
| VN1261 | 0.000158072 |
| VN1262 | 0 |
| VN1263 | 8.53E-05 |
| VN1264 | 0.000165878 |
| VN1265 | 0.000119173 |
| VN1266 | 9.13E-05 |
| VN1267 | 0 |
| VN1268 | 0 |
| VN1269 | 7.46E-05 |
| VN1270 | 0.000107547 |
| VN1271 | 8.83E-05 |
| VN1272 | 0 |
| VN1273 | 8.55E-05 |
| VN1274 | 0.000137628 |
| VN1275 | 0 |
| VN1276 | 0 |
| VN1277 | 0.000608855 |
| VN1279 | 0 |
| VN1280 | 9.86E-05 |
| VN1282 | 8.80E-05 |
| VN1283 | 0.000243597 |
| VN1284 | 0 |
| VN1285 | 0 |
| VN1286 | 7.89E-05 |
| VN1287 | 9.96E-05 |
| VN1288 | 0 |
| VN1290 | 0.00010972 |
| VN1291 | 0 |
| VN1292 | 9.25E-05 |
| VN1293 | 0.000112919 |
| VN1294 | 8.50E-05 |
| VN1295 | 0.000507853 |
| VN1296 | 0 |
| VN1297 | 7.27E-05 |
| VN1298 | 0 |
| VN1299 | 0.000165889 |
| VN1300 | 0.002475928 |
| VN1301 | 0.000217382 |
| VN1302 | 0 |
| VN1307 | 0 |
| VN1308 | 0 |
| VN1309 | 0 |

### Supplementary Table 1 | The characteristics of CHD samples and control samples in the study

| **Characteristics** | **CHD patients** | **Control subjects** | ***p**.**value**** | **permuted *p.value*** |
|---|---|---|---|---|
| Sample number | 59 | 43 | - | - |
| Age (median, range), year | 61, 35-79 | 59, 50-70 | 0.07 | 0.12 |
| CKMB (median, range), U/L | 7.40, 0.50-32.60 | 7.13, 2.30-14.70 | 0.15 | 0.21 |
| ALB (median, range), g/L | 37.90, 26.30-47.20 | 40.68, 35.40-44.10 | 4.06E-05‡ | 8.40E-03‡ |
| ALT (median, range), U/L | 26, 7-86 | 24.47, 10.00-56.00 | 0.02‡ | 0.02‡ |
| TP (median, range), g/L | 64.80, 55.60-75.10 | 73.52, 66.70-82.10 | 1.10E-14‡ | 1.00E-04‡ |
| AST (median, range), U/L | 24.30, 14.95-77.50 | 26.48, 19.00-45.00 | 0.17 | 0.18 |
| CREA (median, range), µmol/L | 84.20, 47.00-288.00 | 80.19, 50.00-123.00 | 0.09 | 0.48 |
| HBDH (median, range), U/L | 132, 91-544 | 151.48, 94.00-206.00 | 0.47 | 0.48 |
| TRIG (median, range), mmol/L | 1.57, 0.67-4.83 | 1.79, 0.37-5.09 | 0.96 | 0.18 |
| LDLC (median, range), mmol/L | 2.83, 0.87-4.99 | 3.32, 1.79-5.64 | 0.01‡ | 0.01‡ |
| CHOL (median, range), mmol/L | 4.48, 2.42-7.26 | 5.46, 3.08-8.26 | 4.69E-05‡ | 1.00E-04‡ |
| HDLC (median, range), mmol/L | 1.01, 0.66-1.59 | 1.32, 0.90-2.21 | 1.25E-07‡ | 1.00E-04‡ |
| APOB (median, range), g/L | 0.81, 0.52-1.21 | 0.92, 0.59-1.38 | 1.17E-03‡ | 5.00E-04‡ |
| APOA (median, range), g/L | 1.06, 0.56-1.90 | 1.30, 0.83-2.33 | 0.01‡ | 8.10E-03‡ |
| LPA (median, range), mg/L | 123.24, 23.93-1386.11 | 240.68, 18.00-678.00 | 0.92 | 0.48 |

| | | | | |
|---|---|---|---|---|
| CKMB, creatine kinase MB; ALB, albumin; ALT, Alanine aminotransferase; TP, Total Protein; AST, Aspartate transaminase; CREA, creatinine; HBDH, hydroxybutyrate dehydrogenase; TRIG, triglyceride; LDLC, low-density lipoprotein; CHOL, cholesterol; HDLC, high-density lipoprotein; APOB, apolipoprotein (b); APOA, apolipoprotein (a); LPA, lipoprotein (a). * *P.value* calculated by Student's t-test. †Permuted *p.value* calculated by PERMANOVA analysis (permutation=1000), permuted *p.value* less than 0.05 indicated significant impacts on metabolic profiles. I Significant clinical biochemical indicators affected metabolic profiles of CHD patients (*p.value* and permutated *p.value* less than 0.05) | | | | |

**Table 2 | Potential urine biomarkers for discriminating CHD patients from control subjects**

| **m/z** | **RT(min)*** | **FC(CHD/** | **Adjusted *p.valu*e‡** | ***VIP*§** | **Adduct ion** | **Formula** | **Metabolites** | **Pathways** |
|---|---|---|---|---|---|---|---|---|
| | | **control)†** | | | | | | |
| 132.07 | 1.9 | 0.41 | 2.36E-02 | 1.41 | H+ | C₄H₉N₃O₂ | Creatine# | Arginine and proline metabolism |
| 205.06 | 1.79 | 8.45 | 9.69E-12 | 2.87 | Na+ | C₆H₁₄O₆ | Mannitol# | Fructose and mannose metabolism |
| 318.29 | 11.37 | 0.39 | 1.65E-05 | 2.51 | H+ | C₁₈H₃₉NO₃ | Phytosphingosine# | Sphingolipid metabolism |
| 324.04 | 10.25 | 165.99 | 4.28E-14 | 2.3 | Na+ | C₈H₁₆NO₉P | N-Acetyl-D-glucosamine 6-phosphate!# | Amino sugar and nucleotide sugar metabolism |

| | | | | | | | | |
|---|---|---|---|---|---|---|---|---|
| *Retention time.†Fold change (FC=mean of metabolite ion intensity in CHD patients/ mean of metabolite ion intensity in healthy controls). When FC was larger than 1, it represented that the biomarker was enriched in CHD patients. When FC was lower than 1, it represented that the biomarker was enriched in healthy individuals .‡Adjusted *p.value* calculated by the two-tailed Wilcoxon rank-sum tests after false discovery rate correction.§VIP (Variable Importance for Projection), one indicator reflecting the capability of the variables to explain Y.#Metabolites matched with commercial available reference standards.^Metabolites which have matched characteristic peaks but mismatched retention time with commercial available reference standards. | | | | | | | | |

### Supplementary Table 2 | Seven common potential biomarkers in plasma and urine samples

| **Category** | **m/z** | **RT(min)*** | **FC(CHD/ control)†** | **Adjusted *p.value*‡** | ***VIP§*** | **NAME** | **PATHWAYS** |
|---|---|---|---|---|---|---|---|
| Potential biomarkers in plasma | 185.04 | 10.24 | 0.33 | 3.69E-07 | 1.57 | unknown | unknown |
| | 311.05 | 10.46 | 29.38 | 1.94E-13 | 2.34 | unknown | unknown |
| | 310.04 | 10.46 | 28.41 | 1.65E-13 | 2.35 | unknown | unknown |
| | 309.05 | 10.46 | 26.40 | 9.08E-14 | 2.35 | unknown | unknown |
| | 202.04 | 9.72 | 0.38 | 2.70E-04 | 1.41 | unknown | unknown |
| | 324.04 | 9.33 | 8.58 | 2.08E-12 | 2.16 | N-Acetyl-D-glucosamine 6-phosphate∥¶ | Amino sugar and nucleotide sugar metabolism |
| | 125.01 | 10.46 | 7.86 | 1.50E-11 | 2.31 | unknown | unknown |
| | 185.04 | 10.30 | 0.34 | 1.70E-03 | 1.62 | unknown | unknown |
| | 311.05 | 10.51 | 59.41 | 3.75E-14 | 2.26 | unknown | unknown |
| | 310.04 | 11.25 | 110.51 | 5.70E-14 | 2.47 | unknown | unknown |
| Potential biomarkers in urine | 309.05 | 11.28 | 12.65 | 1.28E-07 | 2.03 | unknown | unknown |
| | 202.04 | 9.77 | 0.58 | 8.10E-04 | 1.06 | unknown | unknown |
| | 324.04 | 10.25 | 165.99 | 4.28E-14 | 2.30 | N-Acetyl-D-glucosamine 6-phosphate∥¶ | Amino sugar and nucleotide sugar metabolism |
| | 125.01 | 11.18 | 158.93 | 5.11E-14 | 2.42 | unknown | unknown |

| | | | | | | | |
|---|---|---|---|---|---|---|---|
| * Retention time. † Fold change (FC=mean of metabolite ion intensity in CHD patients/ mean of metabolite ion intensity in healthy controls). When FC was larger than 1, it represented that the biomarker was enriched in CHD patients. When FC was lower than 1, it represented that the biomarker was enriched in healthy individuals. ‡ Adjusted *p.value* calculated by the two-tailed Wilcoxon rank-sum tests after false discovery rate correction. §VIP (Variable Importance for Projection), one indicator reflecting the capability of the variables to explain Y. ∥ Metabolites matched with commercial available reference standards. ¶ Metabolites matched characteristic peaks but mismatching retention time with commercial available reference standards. | | | | | | | |

### Supplementary Table 3 | Spearman correlation analysis of the 7 common metabolites

| **Plasma** | **Urine** | **Coefficient** | ***p**.**value**** | **Adjusted *p.value*** | **Enrichment** |
|---|---|---|---|---|---|
| 311.05 | 311.05 | 0.812 | 3.18E-25 | 3.12E-24 | CHD |
| 310.04 | 310.04 | 0.803 | 3.00E-24 | 1.63E-23 | CHD |
| 324.04 | 324.04 | 0.747 | 1.83E-19 | 5.60E-19 | CHD |
| 125.01 | 125.01 | 0.687 | 1.38E-15 | 3.22E-15 | CHD |
| 202.04 | 202.04 | 0.642 | 0 | 0 | Control |
| 309.05 | 309.05 | 0.556 | 1.96E-09 | 4.18E-09 | CHD |
| 185.04 | 185.04 | 0.547 | 4.48E-09 | 8.78E-09 | Control |

| | | | | | |
|---|---|---|---|---|---|
| * *P.value* calculated by spearman correlation analysis. †Adjusted *p.value* calculated by false discovery rate correction. | | | | | |

**Table 4 | AUC results of urine training and validation datasets**

| **Name** | **Urine training datasets** | | | **Urine validation datasets** | | |
|---|---|---|---|---|---|---|
| | ***AUC*** 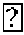 | ***p.value* †** | **FC(CHD/control) ‡** | ***AUC*** 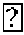 | ***p.value* †** | **FC(CHD/control) ‡** |
| N-Acetyl-D-glucosamine 6-phosphate | 0.96 | 4.44E-09 | 165.99 | 0.88 | 8.29E-16 | 36.91 |
| Mannitol | 0.92 | 2.11E-11 | 8.45 | 0.81 | 3.99E-08 | 2.62 |
| Creatine | 0.66 | 2.85E-02 | 0.41 | 0.57 | 7.59E-01 | 0.94 |
| Phytosphingosine | 0.78 | 1.55E-05 | 0.39 | 0.55 | 2.31E-01 | 1.05 |

| | | | | | | |
|---|---|---|---|---|---|---|
| **AUC* calculated by online tool - ROCCET (http://www.roccet.ca). †*P.value* calculated by T-test. ‡*Fold change.* | | | | | | |

### Supplementary Table 4 | Spearman correlation analysis of clinical data and identified biomarkers

| **Potential Biomarkers** | **Clinical biochemical indicators** | **Coefficient** | ***p.vlaue**** | **Adjusted *p.value* t** |
|---|---|---|---|---|
| LysoPC(18:0) | CHOL | 0.52 | 2.39E-08 | 7.89E-07 |
| LysoPC(P-16:0) | HDLC | 0.56 | 8.45E-10 | 7.39E-08 |
| LysoPC(18:0) | HDLC | 0.55 | 2.56E-09 | 1.29E-07 |
| LysoPC(14:0) | TP | 0.59 | 5.19E-11 | 1.71E-08 |
| LysoPC(18:0) | TP | 0.57 | 3.13E-10 | 5.16E-08 |
| LysoPC(16:1(9Z)) | TP | 0.56 | 9.08E-10 | 7.39E-08 |
| LysoPC(15:0) | TP | 0.56 | 1.12E-09 | 7.39E-08 |
| 1-Palmitoylglycerophosphocholine | TP | 0.55 | 2.74E-09 | 1.29E-07 |
| LysoPC(18:3(9Z,12Z,15Z)) | TP | 0.54 | 6.40E-09 | 2.64E-07 |
| LysoPC(20:3(5Z,8Z,11Z)) | TP | 0.53 | 8.31E-09 | 3.05E-07 |
| LysoPC(18:4(6Z,9Z,12Z,15Z)) | TP | 0.51 | 3.63E-08 | 1.09E-06 |

| | | | | |
|---|---|---|---|---|
| ** P.value* calculated by spearman correlation analysis. tadjusted *p.value* calculated by false discovery rate correction. | | | | |

### Supplementary Table 6 | Spearman correlation analysis of 65 KOs and identified biomarkers

| KO | *KO-p.value** | KO-enrich | metabolites | Coefficient | *p.value*† | *q.value* |
|---|---|---|---|---|---|---|
| K01443 | 1.87E-02 | 1 | N-Acetyl-D-glucosamine-6-phosphate(plasma) | 0.28 | 4.16E-03 | 1.56E-02 |
| K01788 | 5.48E-04 | 1 | N-Acetyl-D-glucosamine-6-phosphate(plasma) | 0.39 | 5.55E-05 | 2.71E-03 |
| K01443 | 1.87E-02 | 1 | N-Acetyl-D-glucosamine-6-phosphate(urine) | 0.26 | 7.17E-03 | 2.15E-02 |
| K01788 | 5.48E-04 | 1 | N-Acetyl-D-glucosamine-6-phosphate(urine) | 0.31 | 1.40E-03 | 8.56E-03 |
| K02755 | 1.04E-03 | 1 | Mannitol | 0.36 | 2.63E-04 | 5.11E-03 |
| K02756 | 1.78E-03 | 1 | Mannitol | 0.33 | 6.31E-04 | 6.20E-03 |
| K02759 | 1.52E-03 | 1 | Mannitol | 0.33 | 8.64E-04 | 6.79E-03 |
| K02760 | 7.79E-05 | 1 | Mannitol | 0.33 | 8.48E-04 | 6.79E-03 |
| K02768 | 7.00E-03 | 1 | Mannitol | 0.31 | 1.73E-03 | 9.25E-03 |
| K02769 | 1.53E-02 | 1 | Mannitol | 0.27 | 6.99E-03 | 2.12E-02 |
| K02777 | 4.36E-04 | 1 | Mannitol | 0.32 | 1.09E-03 | 7.10E-03 |
| K02793 | 3.13E-03 | 1 | Mannitol | 0.30 | 2.33E-03 | 1.06E-02 |
| K02794 | 1.87E-02 | 1 | Mannitol | 0.29 | 3.61E-03 | 1.41E-02 |
| K02808 | 1.56E-02 | 1 | Mannitol | 0.24 | 1.52E-02 | 3.71E-02 |
| K02809 | 1.91E-03 | 1 | Mannitol | 0.27 | 6.17E-03 | 2.00E-02 |
| K02818 | 9.43E-04 | 1 | Mannitol | 0.31 | 1.79E-03 | 9.25E-03 |
| K02821 | 6.53E-04 | 1 | Mannitol | 0.32 | 9.40E-04 | 6.79E-03 |
| K11202 | 3.49E-03 | 1 | Mannitol | 0.30 | 2.11E-03 | 9.78E-03 |

| | | | | | | |
|---|---|---|---|---|---|---|
| * KO-*p.value* calculated by the two-tailed Wilcoxon rank-sum tests †*P.value* calculated by spearman correlation analysis. ‡*Q.value* calculated by false discovery rate correction. KO-enrich: 1 represented that the KO was enriched in CHD patients, and 0 represented that the KO was enriched in healthy individuals. | | | | | | |

**Table 5 | Spearman correlation analysis of 512 species and identified biomarkers**

| Species | Species-*p.value** | Species-*q.value*_{†} | Species-enrich | metabolites | Coefficient | *p.value*_{‡} | *q. value*† |
|---|---|---|---|---|---|---|---|
| Clostridium sp. HGF2 | 9.86E-05 | 8.65E-03 | 1 | N-Acetyl-D-glucosamine-6-phosphate(plasma) | 0.31 | 1.31E-03 | 3.57E-02 |
| Clostridium sp. HGF2 | 9.86E-05 | 8.65E-03 | 1 | N-Acetyl-D-glucosamine-6-phosphate(urine) | 0.27 | 6.24E-03 | 4.99E-02 |
| Clostridium sp. HGF2 | 9.86E-05 | 8.65E-03 | 1 | Mannitol(urine) | 0.26 | 8.52E-03 | 4.68E-02 |
| Streptococcus sp. M334 | 3.13E-02 | 2.39E-01 | 1 | Mannitol(urine) | 0.27 | 5.94E-03 | 3.95E-02 |
| Streptococcus sp. M143 | 3.49E-02 | 2.40E-01 | 1 | Mannitol(urine) | 0.2 | 4.26E-02 | 4.72E-02 |

| | | | | | | | |
|---|---|---|---|---|---|---|---|
| * Species-*p.value* calculated by the two-tailed Wilcoxon rank-sum tests tSpecies-*q*. value and *q.value* calculated by false discovery rate correction. ‡*P.value* calculated by spearman correlation analysis. Species-enrich: 1 represented that the species was enriched in CHD patients, and 0 represented that the species was enriched in healthy individuals. | | | | | | | |

Thus the inventors have identified and validated 7 plasma metabolites and 2 urine metabolites for early and non-invasive diagnosis of CHD by a random forest model based on the associated metabolites. And the inventors have constructed a method to evaluate the risk of CHD based on these associated metabolites.

## Claims

1. A method for diagnosis of coronary heart disease (CHD) in a subject, wherein the method comprises the following steps: 1) determining a level of each of the biomarkers of a biomarker composition in a sample from the subject, wherein the biomarker composition comprises N-Acetyl-D-glucosamine 6-phosphate (GlcNAc-6-P) and mannitol, wherein the sample is urine; 2) comparing the level of each of the biomarkers of step 1) with a reference dataset or a reference value.

2. The method according to claim 1, wherein the reference value is a reference value of healthy controls.

3. The method according to claim 1 or 2, wherein the reference dataset comprises the level of each of the biomarkers in samples from CHD patients and healthy controls.

4. The method according to any one of claims 1 to 3, wherein the biomarker composition further comprises one or more selected from creatine and phytosphingosine.

5. The method according to any one of claims 1 to 4, wherein the step 2) further comprises a step of executing a multivariate statistical model to output the probability of illness.

6. The method according to claim 5, wherein the multivariate statistical model is random forest model.

7. The method according to claim 5 or 6, wherein the subject is determined as being at risk of CHD or having CHD if the probability of illness ≥0.5.

8. The method according to any one of claims 1 to 4, wherein, when compared with a reference value, an increase of GlcNAc-6-P and/or mannitol indicates that the subject has CHD, or a decrease of creatine and/or phytosphingosine indicates that the subject has CHD.

9. A method for screening a candidate compound for treatment of coronary heart disease (CHD), wherein the method comprises the following steps: 1) determining a level of each of the biomarkers of a biomarker composition in a sample collected from a non-human subject that has been administered with the candidate compound, wherein the biomarker composition comprises N-Acetyl-D-glucosamine 6-phosphate (GlcNAc-6-P) and mannitol, and the sample is urine; 2) comparing the level of each of the biomarkers of step 1) with the level before administering the candidate compound,
wherein optionally, the biomarker composition further comprises one or more selected from creatine and phytosphingosine.

10. A method for evaluation of the effect of a treatment of coronary heart disease (CHD), wherein the method comprises the following steps: 1) determining a level of each of the biomarkers of a biomarker composition in a sample collected from a subject that has been treated for CHD, wherein the biomarker composition comprises N-Acetyl-D-glucosamine 6-phosphate (GlcNAc-6-P) and mannitol, and the sample is urine; 2) comparing the level of each of the biomarkers of step 1) with the level before the treatment to the subject,
wherein optionally, the biomarker composition further comprises one or more selected from creatine and phytosphingosine.

11. The method according to claim 9 or 10, wherein a decrease of GIcNAc-6-P and/or mannitol indicates that the compound is a candidate compound effective for treatment of CHD in a subject or the treatment of CHD in the subject is effective; or
an increase of creatine and/or phytosphingosine indicates that the compound is a candidate compound effective for treatment of CHD in a subject or the treatment of CHD in the subject is effective.

12. The method according to any one of claims 1, 9 or 10, wherein the step 1) is carried out by mass spectrometry, preferably, mass spectrometry in conjunction with chromatography, such as gas chromatography mass spectrometry (GC-MS) or liquid chromatography mass spectrometry (LC-MS).

13. The method according to any one of claims 1, 9 or 10, wherein the method further comprises a step of processing the sample before step 1).

## Patentansprüche

1. Verfahren zum Diagnostizieren einer koronaren Herzkrankheit (KHK) in einem Subjekt, wobei das Verfahren die folgenden Schritte umfasst: 1) Bestimmen eines Spiegels jedes der Biomarker einer Biomarkerzusammensetzung in einer Probe von dem Subjekt, wobei die Biomarkerzusammensetzung N-Acetyl-D-glucosamin-6-phosphat (GlcNAc-6-P) und Mannit umfasst, wobei die Probe Urin ist; 2) Vergleichen des Spiegels jedes der Biomarker aus Schritt 1) mit einem Referenzdatensatz oder einem Referenzwert.

2. Verfahren nach Anspruch 1, wobei der Referenzwert ein Referenzwert von gesunden Kontrollen ist.

3. Verfahren nach Anspruch 1 oder 2, wobei der Referenzdatensatz den Spiegel jedes der Biomarker in Proben von KHK-Patienten und gesunden Kontrollen umfasst.

4. Verfahren nach einem der Ansprüche 1 bis 3, wobei die Biomarkerzusammensetzung ferner eine oder mehrere umfasst, ausgewählt aus Kreatin und Phytosphingosin.

5. Verfahren nach einem der Ansprüche 1 bis 4, wobei der Schritt 2) ferner einen Schritt des Ausführens eines multivariaten statistischen Modells für die Ausgabe der Wahrscheinlichkeit einer Krankheit umfasst.

6. Verfahren nach Anspruch 5, wobei das multivariate statistische Modell ein Random-Forest-Modell ist.

7. Verfahren nach Anspruch 5 oder 6, wobei das Subjekt als für KHK gefährdet oder mit KHK bestimmt wird, wenn die Krankheitswahrscheinlichkeit ≥0,5 beträgt.

8. Verfahren nach einem der Ansprüche 1 bis 4, wobei ein Anstieg von GlcNAc-6-P und/oder Mannit, wenn mit einem Referenzwert verglichen, anzeigt, dass das Subjekt KHK hat, oder eine Verringerung von Kreatin und/oder Phytosphingosin anzeigt, dass das Subjekt KHK hat.

9. Verfahren zum Screening einer Kandidatenverbindung für die Behandlung einer koronaren Herzkrankheit (KHK), wobei das Verfahren die folgenden Schritte umfasst: 1) Bestimmen eines Spiegels jedes der Biomarker einer Biomarkerzusammensetzung in einer Probe, die von einem nicht menschlichen Subjekt entnommen wurde, dem die Kandidatenverbindung verabreicht wurde, wobei die Biomarkerzusammensetzung N-Acetyl-D-glucosamin-6-phosphat (GlcNAc-6-P) und Mannit umfasst, und die Probe Urin ist; 2) Vergleichen des Spiegels jedes der Biomarker aus Schritt 1) mit dem Spiegel vor der Verabreichung der Kandidatenverbindung,
wobei optional die Biomarkerzusammensetzung ferner eines oder mehrere umfasst, die aus Kreatin und Phytosphingosin ausgewählt sind.

10. Verfahren zum Bewerten der Wirkung einer Behandlung einer koronaren Herzkrankheit (KHK), wobei das Verfahren die folgenden Schritte umfasst: 1) Bestimmen eines Spiegels jedes der Biomarker einer Biomarkerzusammensetzung in einer Probe, die von einem Subjekt entnommen wurde, das für KHK behandelt worden ist, wobei die Biomarkerzusammensetzung N-Acetyl-D-glucosamin-6-phosphat (GlcNAc-6-P) und Mannit umfasst und die Probe Urin ist; 2) Vergleichen des Spiegels jedes der Biomarker aus Schritt 1) mit dem Spiegel vor der Behandlung an das Subjekt,
wobei optional die Biomarkerzusammensetzung ferner eines oder mehrere umfasst, die aus Kreatin und Phytosphingosin ausgewählt sind.

11. Verfahren nach Anspruch 9 oder 10, wobei eine Verringerung von GlcNAc-6-P und/oder Mannit anzeigt, dass die Verbindung eine Kandidatenverbindung ist, die für die Behandlung von KHK in einem Subjekt wirksam ist, oder die Behandlung von KHK in dem Subjekt wirksam ist; oder
ein Anstieg von Kreatin und/oder Phytosphingosin anzeigt, dass die Verbindung eine Kandidatenverbindung ist, die für die Behandlung von KHK in einem Subjekt wirksam ist, oder die Behandlung von KHK in dem Subjekt wirksam ist.

12. Verfahren nach einem der Ansprüche 1, 9 oder 10, wobei der Schritt 1) durch Massenspektrometrie, vorzugsweise Massenspektrometrie in Verbindung mit Chromatographie, wie Gaschromatographie-Massenspektrometrie (GC-MS) oder Flüssigchromatographie-Massenspektrometrie (LC-MS) ausgeführt wird.

13. Verfahren nach einem der Ansprüche 1, 9 oder 10, wobei das Verfahren ferner einen Schritt des Verarbeitens der Probe vor Schritt 1) umfasst.

## Revendications

1. Procédé de diagnostic d'une maladie coronarienne (CHD) chez un sujet, dans lequel le procédé comprend les étapes suivantes : 1) la détermination d'un taux de chacun des biomarqueurs d'une composition de biomarqueurs dans un échantillon du sujet, dans lequel la composition de biomarqueurs comprend le N-acétyl-D-glucosamine 6-phosphate (GlcNAc-6-P) et le mannitol, dans lequel l'échantillon est de l'urine ; 2) la comparaison du taux de chacun des biomarqueurs de l'étape 1) avec un ensemble de données de référence ou une valeur de référence.

2. Procédé selon la revendication 1, dans lequel la valeur de référence est une valeur de référence de témoins sains.

3. Procédé selon la revendication 1 ou 2, dans lequel l'ensemble de données de référence comprend le taux de chacun des biomarqueurs dans des échantillons de patients atteints de CHD et de témoins sains.

4. Procédé selon l'une quelconque des revendications 1 à 3, dans lequel la composition de biomarqueurs comprend en outre un ou plusieurs éléments sélectionnés parmi la créatine et la phytosphingosine.

5. Procédé selon l'une quelconque des revendications 1 à 4, dans lequel l'étape 2) comprend en outre une étape d'exécution d'un modèle statistique multivarié afin d'obtenir la probabilité de maladie.

6. Procédé selon la revendication 5, dans lequel le modèle statistique multivarié est un modèle à forêt aléatoire.

7. Procédé selon la revendication 5 ou 6, dans lequel le sujet est déterminé comme présentant un risque de CHD ou étant atteint de CHD si la probabilité de maladie est ≥ 0,5.

8. Procédé selon l'une quelconque des revendications 1 à 4, dans lequel, par comparaison avec une valeur de référence, une augmentation du GlcNAc-6-P et/ou du mannitol indique que le sujet est atteint de CHD, ou une diminution de créatine et/ou de phytosphingosine indique que le sujet est atteint de CHD.

9. Procédé de dépistage d'un composé candidat pour un traitement d'une maladie coronarienne (CHD), dans lequel le procédé comprend les étapes suivantes : 1) la détermination d'un taux de chacun des biomarqueurs d'une composition de biomarqueurs dans un échantillon prélevé sur un sujet non humain auquel a été administré le composé candidat, dans lequel la composition de biomarqueurs comprend le N-acétyl-D-glucosamine 6-phosphate (GlcNAc-6-P) et le mannitol, et l'échantillon est de l'urine ; 2) la comparaison du taux de chacun des biomarqueurs de l'étape 1) avec le taux avant l'administration du composé candidat,
dans lequel éventuellement, la composition de biomarqueurs comprend en outre un ou plusieurs éléments sélectionnés parmi la créatine et la phytosphingosine.

10. Procédé d'évaluation de l'effet d'un traitement d'une maladie coronarienne (CHD), dans lequel le procédé comprend les étapes suivantes : 1) la détermination d'un taux de chacun des biomarqueurs d'une composition de biomarqueurs dans un échantillon prélevé sur un sujet qui a été traité pour une CHD, dans lequel la composition de biomarqueurs comprend le N-acétyl-D-glucosamine 6-phosphate (GlcNAc-6-P) et le mannitol, et l'échantillon est de l'urine ; 2) la comparaison du taux de chacun des biomarqueurs de l'étape 1) avec le taux avant le traitement du sujet,
dans lequel éventuellement, la composition de biomarqueurs comprend en outre un ou plusieurs éléments sélectionnés parmi la créatine et la phytosphingosine.

11. Procédé selon la revendication 9 ou 10, dans lequel une diminution du GlcNAc-6-P et/ou du mannitol indique que le composé est un composé candidat efficace pour le traitement d'une CHD chez un sujet ou que le traitement d'une CHD chez le sujet est efficace ; ou
une augmentation de la créatine et/ou de la phytosphingosine indique que le composé est un composé candidat efficace pour le traitement d'une CHD chez un sujet ou que le traitement d'une CHD chez le sujet est efficace.

12. Procédé selon l'une quelconque des revendications 1, 9 ou 10, dans lequel l'étape 1) est réalisée par spectrométrie de masse, de préférence par spectrométrie de masse en conjonction avec la chromatographie, telle que la chromatographie en phase gazeuse-spectrométrie de masse (GC-MS) ou la chromatographie en phase liquide-spectrométrie de masse (LC-MS).

13. Procédé selon l'une quelconque des revendications 1, 9 ou 10, dans lequel le procédé comprend en outre une étape de traitement de l'échantillon avant l'étape 1).
